# EUROPEAN PATENT APPLICATION

(11) **EP 4 810 575 A1**
(43) Date of publication of application: **23.09.2026**
(21) Application number: 25382270.4
(22) Date of filing: 21.03.2025
(51) Int. Cl.: C09B 29/01, C09B 29/12, A61P 9/00, A61P 17/00, A61P 35/00, A61P 37/00, C09K 11/06, A61K 31/045, A61K 31/19, A61K 31/215

(54) **REVERSIBLE LIGHT-REGULATED AGONISTS FOR LOCAL AND TEMPORARILY RESTRICTED ACTIVATION OF VITAMIN D RECEPTOR**

(71) Applicant: Consejo Superior De Investigaciones Científicas, 28006 Madrid (ES)
(72) Inventor: LLEBARIA SOLDEVILA, Amadeo, E-08034 Barcelona (ES); SERRA COMAS, María del Carmen, E-08034 Barcelona (ES); ROVIRA ALGANS, Xavier, E-08034 Barcelona (ES); CATENA RUIZ, Juan Lorenzo, E-08034 Barcelona (ES); PANARELLO, Silvia, E-08034 Barcelona (ES)
(74) Representative: ABG Intellectual Property Law, S.L.

(57) **Abstract**

The invention relates to a reversible light-regulated agonists compounds of formula (I), or a pharmaceutical salt thereof, wherein the meanings for the various substituents are as disclosed in the description. Further, the invention relates to its use as a medicament, particularly for the treatment of skin diseases, autoimmune diseases, cancer, disorders of calcium metabolism, neuromuscular disorders, and cardiovascular diseases by local and temporarily restricted activation of Vitamin D receptor.

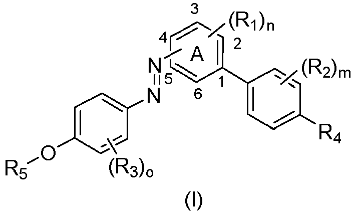

## Description

The invention relates to a reversible light-regulated agonists compounds of formula (I), or a pharmaceutical salt thereof. Further, the invention relates to its use as a medicament, particularly for the treatment of skin diseases, immune diseases, cancer, disorders of calcium metabolism, neuromuscular disorders, and cardiovascular diseases by local and temporarily restricted activation of Vitamin D receptor.

### BACKGROUND ART

Vitamin D receptors (VDR) play very important physiological roles upon activation by the steroid hormone calcitriol (1α,25-Dihydroxyvitamin D3). These include the regulation of calcium absorption and bone remineralization, as well as cell proliferation, migration, and differentiation, and some endocrine and immune processes, among others. Indeed, the alteration of the VDR function and the deficiency of Vitamin D3, the precursor of calcitriol, leads to a variety of serious pathologies such as alterations of wounds repair (Oda Y et al. J Invest Dermatol. 138(11):2423-2431), cardiovascular diseases, such as atherosclerosis (Norman P et al. Circ Res. 17;114(2):379-93), bone malformations, osteoporosis, cancer, inflammation and autoimmune diseases, such as psoriasis, vitiligo and diabetes, among others (Nagpal S et al. Endocr Rev. 26(5):662-87). Accordingly, a number of therapeutic applications has been proposed for the use of VDR ligands (Takahashi T et al. Curr Top Med Chem. 6(12):1303-16). However, some degree of separation between beneficial action and calcium-boosting effects has been achieved with VDR ligands but, to date, the separation has been insufficient to allow oral administration for the general treatment of many conditions and diseases.

Light is essential to interconvert 7-dehydrocholesterol into Vitamin D3 in skin cells, which is subsequently hydroxylated into the active natural derivative calcitriol in the liver and kidney. This series of reactions are mediated by enzymes in skin cells. Upon binding to calcitriol, VDR is translocated from the cytosol to the nucleus where it binds to the retinoid-X receptor, forms a heterodimer and interacts with the DNA. Upon recruitment of several cofactors, the whole complex exerts regulatory functions of a large number of genes.

Vitamin D deficiency has demonstrated to increase the risk of several types of cancer and the activation of VDR, either alone or in combination, to be beneficial for the prognosis and survival rates of patients. The types of cancer for which there is evidence in cellular models, animals and in epidemiological studies is numerous, including colorectal, bladder, breast, melanoma and many others (Seraphin GJ et al. Steroid Biochem Mol Biol. 231:106308).

The implication of vitamin D and its receptor VDR in immunoregulation, pregnancy, aging and metabolism, skin diseases, intestinal biology, bone and musculoskeletal regulation, cancer, and other, was discussed in the "The 24th Workshop on Vitamin D held September 7-9, 2022 in Austin". From the different conferences and discussions on the topic, a number of possible aplications of the present invention can be inferred (Meyer MB et al.J Steroid Biochem Mol Biol. 228:106247).

In the particular case of psoriasis, several types of phototherapy have been proposed based on the use of UVB, UVA and the combination of psoralen and UVA (PUVA), among others. Several mechanisms have been proposed for the different types of phototherapy treatments, including the increase of the systemic serum level of vitamin D in psoriasis patients (Brozyna AA et al. Int J Mol Sci. 23(15):8575). However, controversial results have been published about the beneficial effects and the level of efficacy of these therapies. Moreover, although the short-term side effects of phototherapy are generally mild, long treatments may produce more serious consequences, such as pigmentary disorders, photoaging, cataracts, and carcinogenesis (Zhang P et al.; 33(1):173-180). In particular, PUVA treatment has demonstrated better outcomes. However, the side effects of this approach due to the lack of specificity, which might be severe for patients, limit the broad use of this therapy. To solve these limitations, innovative research proposes multimodal treatments that would synergistically combine the effects of phototherapy with those of light-regulated drugs based on photopharmacological approaches (Ciruela F et al. Front Immunol. 13:904762).

Photopharmacology consists of the activation and inactivation of specific target proteins in localized regions using light-controlled drugs. It does not require genetic modification of the organism, but instead involves light-sensitive small molecules that can be validated and approved using standard drug development procedures. Therefore, this strategy can be applied to wild type organisms (Hüll K et al. Chem Rev. 118(21):10710-10747), including humans. Among the different strategies, photoswitching drugs can be reversibly switched on/off between biologically active and inactive forms when illuminated with different light wavelengths (i.e. colors). This strategy is based on the development of drugs containing a photochromic moiety in their structure, of which azobenzene is one of the most commonly used (Kobauri P et al. Angew Chem Int Ed Engl. 24;62(30): e202300681). Indeed, azobenzene-based photoswitchable drugs have been developed targeting membrane receptors and channels, enzymes, major components of the cytoskeleton and nuclear hormone receptors, among others (Velema WA et al. J Am Chem Soc. 2014 Feb 12;136(6):2178-91). This game-changing technology has demonstrated a huge potential for the generation of new biological knowledge both at the molecular and physiological level.

Accordingly, it would be necessary the development of new molecules useful as reversible light-regulated agonists for local and temporarily restricted activation of Vitamin D receptor using wavelengths suitable for phototherapy that would avoid the appearance of secondary effects, such as hypercalcemia.

### SUMMARY OF THE INVENTION

A first aspect of the present invention relates to a compound of formula (I): 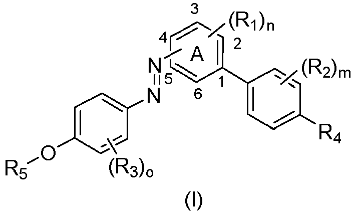 or a pharmaceutical salt thereof, wherein:
the azo group (-N=N-) is linked to any of the available positions 4- or 5- of benzene ring A;
each of n, m and o independently represents an integer from 0 to 2;
each of R₁, R₂ and R₃ independently represents halogen, -C₁-C₄ alkyl, -OR₆, -OCF₃, - CF₃, -N(R₆)₂ or -NO₂;
R₄ represents -(C₁-C₄ alkyl)ₚOR₇, -(C₁-C₄ alkyl)_{q}COOR₈, -(C₁-C₄ alkyl)ᵣCONR₉R₁₀, each of p, q and r independently represents an integer having a value of from 0 to 3;
R₅ represents a linear or branched C₂-C₇-alkyl group substituted with at least one hydroxyl group ;
each R₆ independently represents H or -C₁-C₄ alkyl; and
each R₇;R₈, R₉ and R₁₀ independently represents H or -C₁-C₄-alkyl, wherein said -C₁-C₄-alkyl is optionally substituted by 1 to 3 groups independently selected from the group consisting of -OR₆, -N(R₆)₂, -COOR₆ or -CON(R₆)₂ , or, alternatively, when any one of R₇, R₈, R₉ and R₁₀ is a -C₁-C₄-alkyl substituted by two adjacent groups -OR₆, both -OR₆ groups can be bonded to form an acetal group.

In another embodiment the invention relates to the compound of formula (I) as defined above, wherein the azo group (-N=N-) is linked to position 5- of the benzene ring A.

In another embodiment the invention relates to the compound of formula (I) as defined above, wherein each of n, m and o independently represents an integer having a value of 0 or 1.

In another embodiment the invention relates to the compound of formula (I) as defined above, wherein each of R₁, R₂ and R₃ independently represents a -C₁-C₄ alkyl.

In another embodiment the invention relates to the compound of formula (I) as defined above, wherein each of R₁, R₂ and R₃ independently represents a methyl group or ethyl group; and more preferably wherein each of R₁, R₂ and R₃ independently represents a methyl group.

In another embodiment the invention relates to the compound of formula (I) as defined above, wherein each of p, q and r independently represents an integer having a value of 0 or 1.

In another embodiment the invention relates to the compound of formula (I) as defined above, wherein R₄ represents -(C₁-C₄ alkyl)ₚOR₇ or -(C₁-C₄ alkyl)_{q}COOR₈, and preferably wherein R₄ represents-(C₁-C₄ alkyl)_{q}COOR₈.

In another embodiment the invention relates to the compound of formula (I) as defined above, wherein R₄ is selected from the group consisting of a) -(CH₂)₁₋₂OR₇, wherein R₇ is selected from the group consisting of hydrogen or a group -CH(OR₁₂)CH₂OR₁₂, wherein both R₁₂ are either hydrogen atoms or form together a group -C((CH₃)₂)-, b) -(CH₂)₀₋₁COOR₈ wherein R₈ is selected from the group consisting of hydrogen, methyl or ethyl and c) -CO-NH-CH(CH₂OH)-COOR₁₁, wherein R₁₁ is selected from the group consisting of hydrogen and methyl.

In another embodiment the invention relates to the compound of formula (I) as defined above, wherein:
R₄ represents -(C₁-C₄ alkyl),OR₇ or -(C₁-C₄ alkyl)_{q}COOR₈, and preferably wherein R₄ represents-(C₁-C₄ alkyl)_{q}COOR₈, and
p and q independently represents an integer having a value of from 0 to 1.

In another embodiment the invention relates to the compound of formula (I) as defined above, wherein:
R₄ represents -(C₁-C₄ alkyl)ₚOR₇ or -(C₁-C₄ alkyl)_{q}COOR₈, and preferably wherein R₄ represents-(C₁-C₄ alkyl)_{q}COOR₈, and
R₇ and R₈ independently represents H or -C₁-C₄-alkyl, and preferably H or methyl group, and
p and q independently represents an integer having a value of from 0 to 1.

In another embodiment the invention relates to the compound of formula (I) as defined above, wherein R₄ represents represents -(C₁-C₄ alkyl)_{q}COOR₈.wherein q takes a value of 0 and R₈ is selected from the group consisting of H or methyl.

In another embodiment the invention relates to the compound of formula (I) as defined above, wherein R₄ represents -COOH, -CH₂COOH, -COOMe, -COOEt, -CH₂COOMe, CH₂CH₂OH or CH₂OH, and preferably -COOH, -CH₂COOH, -COOMe, -COOEt, or - CH₂COOMe.

In another embodiment the invention relates to the compound of formula (I) as defined above, wherein R₅ represents a linear or branched C₂-C₇ alkyl substituted with at least one hydroxyl group.

In another embodiment the invention relates to the compound of formula (I) as defined above, wherein R₅ represents a linear or branched C₂-C₇ alkyl substituted with one or two hydroxyl groups, preferably with one hydroxyl group.

In another embodiment the invention relates to the compound of formula (I) as defined above, wherein R₅ represents a linear or branched C₅-C₇ alkyl substituted with one or two hydroxyl groups, preferably with one hydroxyl group.

In another embodiment the invention relates to the compound of formula (I) as defined above, wherein R₅ represents a group selected from the group consisting of 2-hydroxy-3,3-dimethylbutyl, 3-ethyl-3-hydroxypentyl, 3-ethyl-3-hydroxypent-1-en-1-yl and 2-ethyl-2-hydroxybutyl, preferably 2-hydroxy-3,3-dimethylbutyl.

In another embodiment the invention relates to the compound of formula (I) wherein:
each of R₁, R₂ and R₃ independently represents hydrogen or methyl group;
n, m and o are independently 0 or 1;
R₄ is selected from the group consisting of:
   a) -(CH₂)₁₋₂OR₇, wherein R₇ is selected from the group consisting of hydrogen or a group -CH(OR₁₂)CH₂OR₁₂, wherein both R₁₂ are either hydrogen atoms or form together a group -C((CH₃)₂)-,
   b) -(CH₂)₀₋₁COOR₈ wherein R₈ is selected from the group consisting of hydrogen, methyl or ethyl and
   c) -CO-NH-CH(CH₂OH)-COOR₁₁, wherein R₁₁ is selected from the group consisting of hydrogen and methyl; and
R₅ represents a 2-hydroxy-3,3-dimethylbutyl group.

In another embodiment the invention relates to the compound of formula (I) wherein:
each of R₁, R₂ and R₃ independently represents hydrogen or methyl group;
n, m and o are independently 0 or 1;
R₄ is selected -(CH₂)₀₋₁COOR₈ wherein R₈ is selected from the group consisting of hydrogen or methyl and R₅ represents a 2-hydroxy-3,3-dimethylbutyl group.

In another embodiment the invention relates to the compound of formula (I) as defined above, wherein R₈ represents H.

In another embodiment the invention relates to the compound of formula (I) as defined above, wherein the compound of formula (I) is selected from:
Methyl (+/-)-3'-((4-(2-hydroxy-3,3-dimethylbutoxy)-2-methylphenyl)diazenyl)-[1,1'-biphenyl]-4-carboxylate ((+/-)-la-1);
(-)-(R)-Methyl 3'-((4-(2-hydroxy-3,3-dimethylbutoxy)-2-methylphenyl)diazenyl)-[1,1'-biphenyl]-4-carboxylate ((-)-la-1);
(+)-(S)-Methyl 3'-((4-(2-hydroxy-3,3-dimethylbutoxy)-2-methylphenyl)diazenyl)-[1,1'-biphenyl]-4-carboxylate ((+)-la-1);
(+/-)-1-(4-((4'-(hydroxymethyl)-[1,1'-biphenyl]-3-yl)diazenyl)-2-methylphenoxy)-3,3-dimethylbutan-2-ol ((+/-)-la-2);
(+/-)-Methyl 3'-((4-(2-hydroxy-3,3-dimethylbutoxy)-2-methylphenyl)diazenyl)-2-methyl-[1,1'-biphenyl]-4-carboxylate ((+/-)-la-3);
(+/-)-Methyl 3'-((4-(2-hydroxy-3,3-dimethylbutoxy)-3-methylphenyl)diazenyl)-2-methyl-[1,1'-biphenyl]-4-carboxylate ((+/-)-la-4);
(+/-)-3'-((4-(2-Hydroxy-3,3-dimethylbutoxy)-2-methylphenyl)diazenyl)-[1,1'-biphenyl]-4-carboxylic acid ((+/-)-lc-1);
(-)*-(R)* 3'-((4-(2-Hydroxy-3,3-dimethylbutoxy)-2-methylphenyl)diazenyl)-[1,1'-biphenyl]-4-carboxylic acid((-)-Ic-1);
(+)-(S) 3'-((4-(2-Hydroxy-3,3-dimethylbutoxy)-2-methylphenyl)diazenyl)-[1,1'-biphenyl]-4-carboxylic acid ((+)-lc-1);
(+/-)-3'-((4-(2-Hydroxy-3,3-dimethylbutoxy)-3-methylphenyl)diazenyl)-[1,1'-biphenyl]-4-carboxylic acid ((+/-)-lc-2);
(+/-)-3'-((4-(2-Hydroxy-3,3-dimethylbutoxy)-2-methylphenyl)diazenyl)-2-methyl-[1,1'-biphenyl]-4-carboxylic acid ((+/-)-Ic-3);
(+/-)-3'-((4-(2-Hydroxy-3,3-dimethylbutoxy)-3-methylphenyl)diazenyl)-2-methyl-[1,1'-biphenyl]-4-carboxylic acid ((+/-)-Ic-4);
(+/-)-1-(4-((4'-(Hydroxymethyl)-[1,1'-biphenyl]-3-yl)diazenyl)-3-methylphenoxy)-3,3-dimethylbutan-2-ol ((+/-)-Ib-1);
(-)-1-(4-((4'-(Hydroxymethyl)-[1,1'-biphenyl]-3-yl)diazenyl)-3-methylphenoxy)-3,3-dimethylbutan-2-ol ((-)-Ib-1);
(+)-1-(4-((4'-(Hydroxymethyl)-[1,1'-biphenyl]-3-yl)diazenyl)-3-methylphenoxy)-3,3-dimethylbutan-2-ol ((+)-lb-1);
(+/-)-1-(4-((4'-(Hydroxymethyl)-[1,1'-biphenyl]-3-yl)diazenyl)-2-methylphenoxy)-3,3-dimethylbutan-2-ol ((+/-)-lb-2);
(-)-1-(4-((4'-(Hydroxymethyl)-[1,1'-biphenyl]-3-yl)diazenyl)-2-methylphenoxy)-3,3-dimethylbutan-2-ol ((-)-Ib-2);
(+)-1-(4-((4'-(Hydroxymethyl)-[1,1'-biphenyl]-3-yl)diazenyl)-2-methylphenoxy)-3,3-dimethylbutan-2-ol ((+)-lb-2);
(+/-)-1-(4-((4'-(Hydroxymethyl)-2'-methyl-[1,1'-biphenyl]-3-yl)diazinyl)-3-methylphenoxy)-3,3-dimethylbutan-2-ol ((+/-)-Ib-3);
(+/-)-1-(4-((4'-(Hydroxymethyl)-2'-methyl-[1,1'-biphenyl]-3-yl)diazenyl)-2-methylphenoxy)-3,3-dimethylbutan-2-ol ((+/-)-Ib-4);
Methyl-(3'-(4-(2-hydroxy-3,3-dimethylbutoxy)-3-methylphenyl)diazenyl)-[1,1'-biphenyl]-4-carbonyl)-L-serinate (Id-1);
(3'-(4-(2-Hydroxy-3,3-dimethylbutoxy)-3-methylphenyl)diazenyl)-[1,1'-biphenyl]-4-carbonyl)-L-serine) (Id-2);
1-(4-((4'-(((2,2-dimethyl-1,3-dioxolan-4-yl)methoxy)methyl)-[1,1'-biphenyl]-3-yl)diazinyl)-3-methylphenoxy)-3,3-dimethylbutan-2-ol (le-1);
1-(4-((4'-(((2,2-dimethyl-1,3-dioxolan-4-yl)methoxy)methyl)-[1,1'-biphenyl]-3-yl)diazenyl)-2-methylphenoxy)-3,3-dimethylbutan-2-ol (le-2);
3-((3'-((4-(2-hydroxy-3,3-dimethylbutoxy)-2-methylphenyl)diazenyl)-[1,1'-biphenyl]-4-yl)methoxy)propane-1,2-diol (le-3);
3-((3'-((4-(2-hydroxy-3,3-dimethylbutoxy)-3-methylphenyl)diazenyl)-[1,1'-biphenyl]-4-yl)methoxy)propane-1,2-diol (le-4);
(+/-)-Ethyl 4'-((4-(2-hydroxy-3,3-dimethylbutoxy)-2-methylphenyl)diazenyl)-[1,1'-biphenyl]-4-carboxylate ((+/-)-la-5);
(+/-)-Ethyl 4'-((4-(2-hydroxy-3,3-dimethylbutoxy)-3-methylphenyl)diazenyl)-[1,1'-biphenyl]-4-carboxylate ((+/-)-la-6);
(+/-)-Methyl 4'-((4-(2-hydroxy-3,3-dimethylbutoxy)-2-methylphenyl)diazenyl)-2-methyl-[1,1'-biphenyl]-4-carboxylate ((+/-)-la-7);
(+/-)-Ethyl 4'-((4-(2-Hydroxy-3,3-dimethylbutoxy)-2-methylphenyl)diazenyl)-3'-methyl-[1,1'-biphenyl]-4-carboxylate ((+/-)-la-8);
(+/-)-Ethyl 4'-((4-(2-hydroxy-3,3-dimethylbutoxy)-3-methylphenyl)diazenyl)-3'-methyl-[1,1'-biphenyl]-4-carboxylate ((+/-)-la-9);
(+/-)-4'-((4-(2-hydroxy-3,3-dimethylbutoxy)-2-methylphenyl)diazenyl)-[1,1'-biphenyl]-4-carboxylic acid ((+/-)-Ic-5);
(+/-)-4'-((4-(2-Hydroxy-3,3-dimethylbutoxy)-3-methylphenyl)diazenyl)-[1,1'-biphenyl]-4-carboxylic acid ((+/-)-Ic-6);
(+/-)-4'-((4-(2-Hydroxy-3,3-dimethylbutoxy)-2-methylphenyl)diazenyl)-3'-methyl-[1,1'-biphenyl]-4-carboxylic acid ((+/-)-Ic-7);
(+/-)-4'-((4-(2-Hydroxy-3,3-dimethylbutoxy)-3-methylphenyl)diazenyl)-3'-methyl-[1,1'-biphenyl]-4-carboxylic acid ((+/-)-Ic-8);
(+/-)-1-(4-((4'-(Hydroxymethyl)-[1,1'-biphenyl]-4-yl)diazenyl)-3-methylphenoxy)-3,3-dimethylbutan-2-ol ((+/-)-Ib-5);
(+/-)-1-(4-((4'-(Hydroxymethyl)-[1,1'-biphenyl]-4-yl)diazenyl)-2-methylphenoxy)-3,3-dimethylbutan-2-ol ((+/-)-Ib-6);
(+/-)-1-(4-((4'-(Hydroxymethyl)-2'-methyl-[1,1'-biphenyl]-4-yl)diazenyl)-3-methylphenoxy)-3,3-dimethylbutan-2-ol ((+/-)-Ib-7);
(+/-)-1-(4-((4'-(Hydroxymethyl)-3-methyl-[1,1'-biphenyl]-4-yl)diazenyl)-2-methylphenoxy)-3,3-dimethylbutan-2-ol ((+/-)-Ib-8);
(+/-)-1-(4-((4'-(Hydroxymethyl)-3-methyl-[1,1'-biphenyl]-4-yl)diazenyl)-3-methylphenoxy)-3,3-dimethylbutan-2-ol ((+/-)-lb-9);
(+/-)-2-(4'-((4-(2-Hydroxy-3,3-dimethylbutoxy)-2-methylphenyl)diazenyl)-3'-methyl-[1,1'-biphenyl]-4-yl)acetic acid ((+/-)-Ic-9);
(+/-)-2-(4'-((4-(2-Hydroxy-3,3-dimethylbutoxy)-3-methylphenyl)diazenyl)-3'-methyl-[1,1'-biphenyl]-4-yl)acetic acid ((+/-)-Ic-10);
(+/-)-Methyl 2-(4'-((4-(2-hydroxy-3,3-dimethylbutoxy)-2-methylphenyl)diazenyl)-3'-methyl-[1,1'-biphenyl]-4-yl)acetate (I(+/-)-a-10);
(+/-)-Methyl 2-(4'-((4-(2-hydroxy-3,3-dimethylbutoxy)-3-methylphenyl)diazenyl)-3'-methyl-[1,1'-biphenyl]-4-yl)acetate ((+/-)-la-11);
(+/-)-1-(4-((4'-(2-hydroxyethyl)-3-methyl-[1,1'-biphenyl]-4-yl)diazinyl)-3-methylphenoxy)-3,3-dimethylbutan-2-ol ((+/-)-lb-10); and
(+/-)-1-(4-((4'-(2-Hydroxyethyl)-3-methyl-[1,1'-biphenyl]-4-yl)diazenyl)-2-methylphenoxy)-3,3-dimethylbutan-2-ol ((+/-)-lb-11).

In another embodiment the invention relates to the compound of formula (I) as defined above, wherein the compound of formula (I) is selected from:
Methyl (+/-)-3'-((4-(2-hydroxy-3,3-dimethylbutoxy)-2-methylphenyl)diazenyl)-[1,1'-biphenyl]-4-carboxylate ((+/-)-la-1);
(-)-(R)-Methyl 3'-((4-(2-hydroxy-3,3-dimethylbutoxy)-2-methylphenyl)diazenyl)-[1,1'-biphenyl]-4-carboxylate ((-)-la-1);
(+)-(S)-Methyl 3'-((4-(2-hydroxy-3,3-dimethylbutoxy)-2-methylphenyl)diazenyl)-[1,1'-biphenyl]-4-carboxylate ((+)-la-1);
(+/-)-1-(4-((4'-(hydroxymethyl)-[1,1'-biphenyl]-3-yl)diazenyl)-2-methylphenoxy)-3,3-dimethylbutan-2-ol ((+/-)-la-2);
(+/-)-Methyl 3'-((4-(2-hydroxy-3,3-dimethylbutoxy)-2-methylphenyl)diazenyl)-2-methyl-[1,1'-biphenyl]-4-carboxylate ((+/-)-la-3);
(+/-)-Methyl 3'-((4-(2-hydroxy-3,3-dimethylbutoxy)-3-methylphenyl)diazenyl)-2-methyl-[1,1'-biphenyl]-4-carboxylate ((+/-)-la-4);
(+/-)-3'-((4-(2-Hydroxy-3,3-dimethylbutoxy)-2-methylphenyl)diazenyl)-[1,1'-biphenyl]-4-carboxylic acid ((+/-)-lc-1);
(-)-(*R)* 3'-((4-(2-Hydroxy-3,3-dimethylbutoxy)-2-methylphenyl)diazenyl)-[1,1'-biphenyl]-4-carboxylic acid((-)-Ic-1);
(+)-(S) 3'-((4-(2-Hydroxy-3,3-dimethylbutoxy)-2-methylphenyl)diazenyl)-[1,1'-biphenyl]-4-carboxylic acid ((+)-lc-1);
(+/-)-3'-((4-(2-Hydroxy-3,3-dimethylbutoxy)-3-methylphenyl)diazenyl)-[1,1'-biphenyl]-4-carboxylic acid ((+/-)-lc-2);
(+/-)-3'-((4-(2-Hydroxy-3,3-dimethylbutoxy)-2-methylphenyl)diazenyl)-2-methyl-[1,1'-biphenyl]-4-carboxylic acid ((+/-)-Ic-3);
(+/-)-3'-((4-(2-Hydroxy-3,3-dimethylbutoxy)-3-methylphenyl)diazenyl)-2-methyl-[1,1'-biphenyl]-4-carboxylic acid ((+/-)-Ic-4);
(+/-)-1-(4-((4'-(Hydroxymethyl)-[1,1'-biphenyl]-3-yl)diazenyl)-3-methylphenoxy)-3,3-dimethylbutan-2-ol ((+/-)-Ib-1);
(-)-1-(4-((4'-(Hydroxymethyl)-[1,1'-biphenyl]-3-yl)diazenyl)-3-methylphenoxy)-3,3-dimethylbutan-2-ol ((-)-Ib-1);
(+)-1-(4-((4'-(Hydroxymethyl)-[1,1'-biphenyl]-3-yl)diazenyl)-3-methylphenoxy)-3,3-dimethylbutan-2-ol ((+)-lb-1);
(+/-)-1-(4-((4'-(Hydroxymethyl)-[1,1'-biphenyl]-3-yl)diazenyl)-2-methylphenoxy)-3,3-dimethylbutan-2-ol ((+/-)-lb-2);
(-)-1-(4-((4'-(Hydroxymethyl)-[1,1'-biphenyl]-3-yl)diazenyl)-2-methylphenoxy)-3,3-dimethylbutan-2-ol ((-)-Ib-2);
(+)-1-(4-((4'-(Hydroxymethyl)-[1,1'-biphenyl]-3-yl)diazenyl)-2-methylphenoxy)-3,3-dimethylbutan-2-ol ((+)-lb-2);
(+/-)-1-(4-((4'-(Hydroxymethyl)-2'-methyl-[1,1'-biphenyl]-3-yl)diazinyl)-3-methylphenoxy)-3,3-dimethylbutan-2-ol ((+/-)-Ib-3);
(+/-)-1-(4-((4'-(Hydroxymethyl)-2'-methyl-[1,1'-biphenyl]-3-yl)diazenyl)-2-methylphenoxy)-3,3-dimethylbutan-2-ol ((+/-)-Ib-4);
Methyl-(3'-(4-(2-hydroxy-3,3-dimethylbutoxy)-3-methylphenyl)diazenyl)-[1,1'-biphenyl]-4-carbonyl)-L-serinate (Id-1);
(3'-(4-(2-Hydroxy-3,3-dimethylbutoxy)-3-methylphenyl)diazenyl)-[1,1'-biphenyl]-4-carbonyl)-L-serine) (Id-2);
1-(4-((4'-(((2,2-dimethyl-1,3-dioxolan-4-yl)methoxy)methyl)-[1,1'-biphenyl]-3-yl)diazinyl)-3-methylphenoxy)-3,3-dimethylbutan-2-ol (le-1);
1-(4-((4'-(((2,2-dimethyl-1,3-dioxolan-4-yl)methoxy)methyl)-[1,1'-biphenyl]-3-yl)diazenyl)-2-methylphenoxy)-3,3-dimethylbutan-2-ol (le-2);
3-((3'-((4-(2-hydroxy-3,3-dimethylbutoxy)-2-methylphenyl)diazenyl)-[1,1'-biphenyl]-4-yl)methoxy)propane-1,2-diol (le-3); and
3-((3'-((4-(2-hydroxy-3,3-dimethylbutoxy)-3-methylphenyl)diazenyl)-[1,1'-biphenyl]-4-yl)methoxy)propane-1,2-diol (le-4).

In another embodiment the invention relates to the compound of formula (I) as defined above, wherein the compound of formula (I) is selected from:
(+/-)-3'-((4-(2-Hydroxy-3,3-dimethylbutoxy)-2-methylphenyl)diazenyl)-[1,1'-biphenyl]-4-carboxylic acid ((+/-)-lc-1);
(-)*-(R)* 3'-((4-(2-Hydroxy-3,3-dimethylbutoxy)-2-methylphenyl)diazenyl)-[1,1'-biphenyl]-4-carboxylic acid((-)-Ic-1);
(+)-(S) 3'-((4-(2-Hydroxy-3,3-dimethylbutoxy)-2-methylphenyl)diazenyl)-[1,1'-biphenyl]-4-carboxylic acid ((+)-lc-1);
(+/-)-3'-((4-(2-Hydroxy-3,3-dimethylbutoxy)-3-methylphenyl)diazenyl)-[1,1'-biphenyl]-4-carboxylic acid ((+/-)-lc-2);
(+/-)-3'-((4-(2-Hydroxy-3,3-dimethylbutoxy)-2-methylphenyl)diazenyl)-2-methyl-[1,1'-biphenyl]-4-carboxylic acid ((+/-)-Ic-3);and
(+/-)-3'-((4-(2-Hydroxy-3,3-dimethylbutoxy)-3-methylphenyl)diazenyl)-2-methyl-[1,1'-biphenyl]-4-carboxylic acid ((+/-)-Ic-4).

In the above definitions, the term Cₐ-C_{b} alkyl as a group or part of a group, means a straight or branched alkyl chain, i.e an aliphatic fully saturated hydrocarbon chain, which contains from a to b carbon atoms, and include, among others, the groups methyl, ethyl, propyl, isopropyl, butyl, isobutyl, sec-butyl, *tert-butyl,* pentyl and hexyl.

A halogen group or its abbreviation halo means fluoro, chloro, bromo or iodo.

The present invention includes the enantiomerically pure, racemic or diastereomeric mixture of a compound of formula (I).

The present invention also relates to a pharmaceutical composition that comprises a compound of the present invention (or a pharmaceutically acceptable salt or solvate thereof) and one or more pharmaceutically acceptable excipients. The excipients must be "acceptable" in the sense of being compatible with the other ingredients of the composition and not deleterious to the recipients thereof.

Accordingly, another aspect of the invention relates to a pharmaceutical composition which comprises a compound of formula (I) a pharmaceutically acceptable salt thereof and one or more pharmaceutically acceptable excipients.

The compounds of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

Another aspect of the invention relates to a compound of formula (I) as defined above or a pharmaceutically acceptable salt thereof for use as a medicament.

Another aspect of the invention relates to a compound of formula (I) as defined above or a pharmaceutically acceptable salt thereof for use in the treatment of a disease selected from skin diseases, immune diseases, cancer, disorders of calcium metabolism, neuromuscular disorders, and cardiovascular diseases.

In another embodiment the invention relates to a compound of formula (I) for the use defined above, wherein skin diseases are selected from hard-to-heal wounds, psoriasis, atopic dermatitis, acne, actinic keratosis, alopecia, vitiligo, skin cell damage from mustard vesicants, insufficient sebum secretion, insufficient dermal firmness, insufficient dermal hydration and wrinkles.

In another embodiment the invention relates to a compound of formula (I) for the use defined above, wherein immune diseases are selected from Type I diabetes, Crohn's disease, host-graft rejection, Multiple sclerosis, Psoriatic arthritis, Rheumatoid arthritis, Scleroderma, Systemic lupus erythematosus and Ulcerative colitis.

In another embodiment the invention relates to a compound of formula (I) for the use defined above, wherein cancer is selected from bladder cancer, breast cancer, chemoprevention of cancer, colon cancer, leukemia, myelodysplastic syndrome, prostate cancer and skin cancer.

In another embodiment the invention relates to a compound of formula (I) for the use defined above, wherein disorders of calcium metabolism are selected from bone malformation, osteoporosis, and bone maintenance, bone fracture healing, hypercalcemia, osteomalacia and Renal osteodystrophy.

In another embodiment the invention relates to a compound of formula (I) for the use defined above, wherein cardiovascular diseases are selected from atherosclerosis, cardiac hypertrophy and fibrosis

Another aspect of the invention relates to the use of a compound of formula (I) or a pharmaceutically acceptable salt thereof as a medicament.

Another aspect of the invention relates to the use of a compound of formula (I) or a pharmaceutically acceptable salt thereof for the treatment of a disease selected from skin diseases, autoimmune diseases, cancer, disorders of calcium metabolism, neuromuscular disorders and cardiovascular diseases.

Another aspect of the present invention relates to a method of treating a disease in a subject in need thereof, especially a human being, which comprises administering to said subject an effective amount of a compound of formula (I) or a pharmaceutically acceptable salt thereof.

Another aspect of the present invention relates to a method of treating skin diseases, autoimmune diseases, cancer, disorders of calcium metabolism, neuromuscular disorders and cardiovascular diseases, in a subject in need thereof, especially a human being, which comprises administering to said subject an effective amount of a compound of formula (I) or a pharmaceutically acceptable salt thereof.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### EXAMPLES

Compounds of formula (I) may be synthesized by using some of the methods described below, as well as other processes known in the field of the organic chemistry. Preferred methods include, but are not limited to, the general procedures shown in the following Schemes.

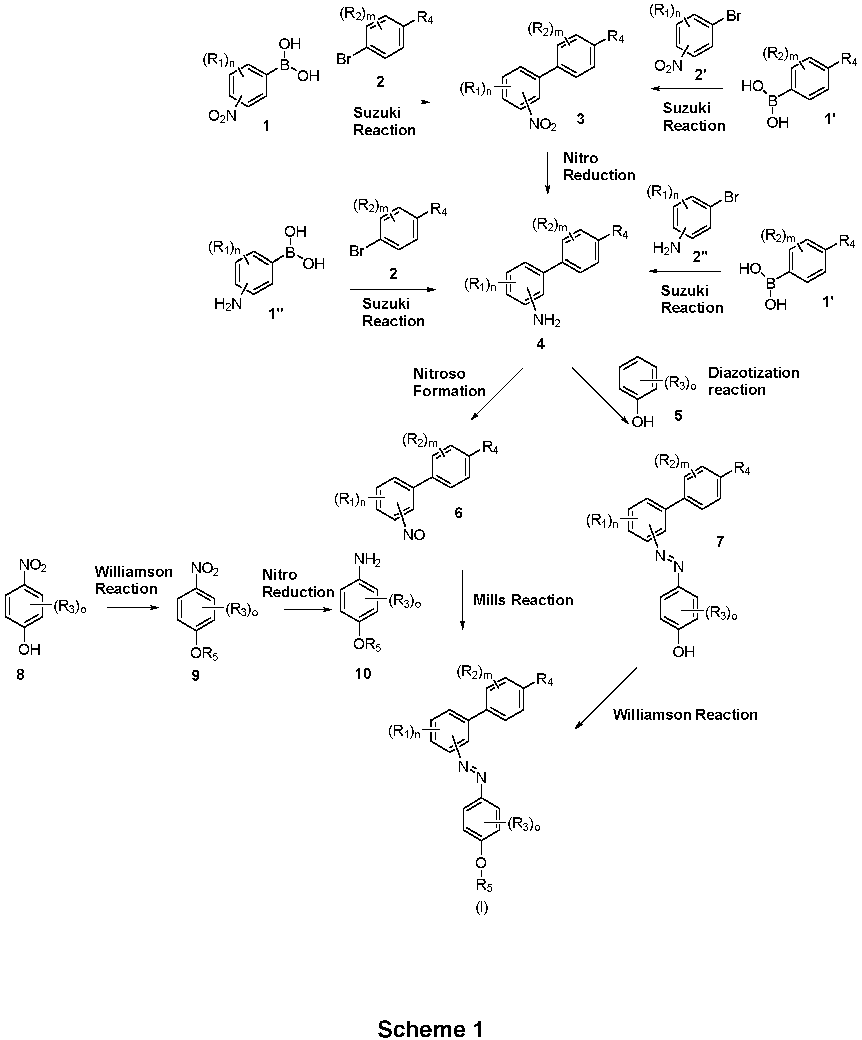

In general, the compounds of the present invention (I) can be obtained by using Mills conditions between the nitrosobiphenyl intermediates type 6 and the amino intermediates type 10 or, alternatively, by a Williamson reaction between phenol intermediates type 7 and a commercial alkylating agent (ex. Halogen derivative or an oxirane). At the same time, amino ethers intermediates type 10 can be prepared by a Williamson reaction between commercial 4-nitrophenols type **8** and an alkylating agent (ex. Halogen derivative or an oxirane), obtaining the nitrobenzene intermediate type 9, which can be converted to intermediate aniline type 10 by reduction of nitro group in standard condition described in the literature (as hydrogenation with Pd/C). On the other hand, both, nitroso intermediates type **6** and phenol intermediates type **7** can be synthetized from a common amino intermediate type **4** by oxidation according to procedures described in the literature such as reaction with Oxone^{®}, or by diazotization in standard condition with a commercial phenol respectively. Finally, the amino intermediate type **4** can be prepared directly by means of a Suzuki reaction between a commercial boronic acid type **1'** or **1"** and a commercial bromobenzene type **2** or **2",** or indirectly by Suzuki reaction between a commercial nitroboronic acid type **1** or **1'** and the a commercial bromobenzene type **2** or **2',** obtaining the biphenylnitro intermediate type **3,** which can be reduced to amine according to procedures described in the literature (as hydrogenation with Pd/C).

Alternatively, in some specific cases where R₂ contains functional groups susceptible to different modifications, these modifications can be carried out on compounds of formula (I) or on intermediates type **4** following methodologies known to experts in the field. Scheme 2 illustrates some of said modifications. In the particular, when R₂ contain an ester group, it can be transformed into an amide, acid, or alcohol following methods described in the literature. When R₂ contain an acid group, it can be also transformed into an amide, ester, alcohol or ester according literature procedures. At the same time, when R₂ contain an alcohol group it can be alkylated via etherification with a suitable alkylating agent. When R₂ contain an acetal group, the corresponding diol could be achieved by hydrolysis of the acetal group in acidic conditions.

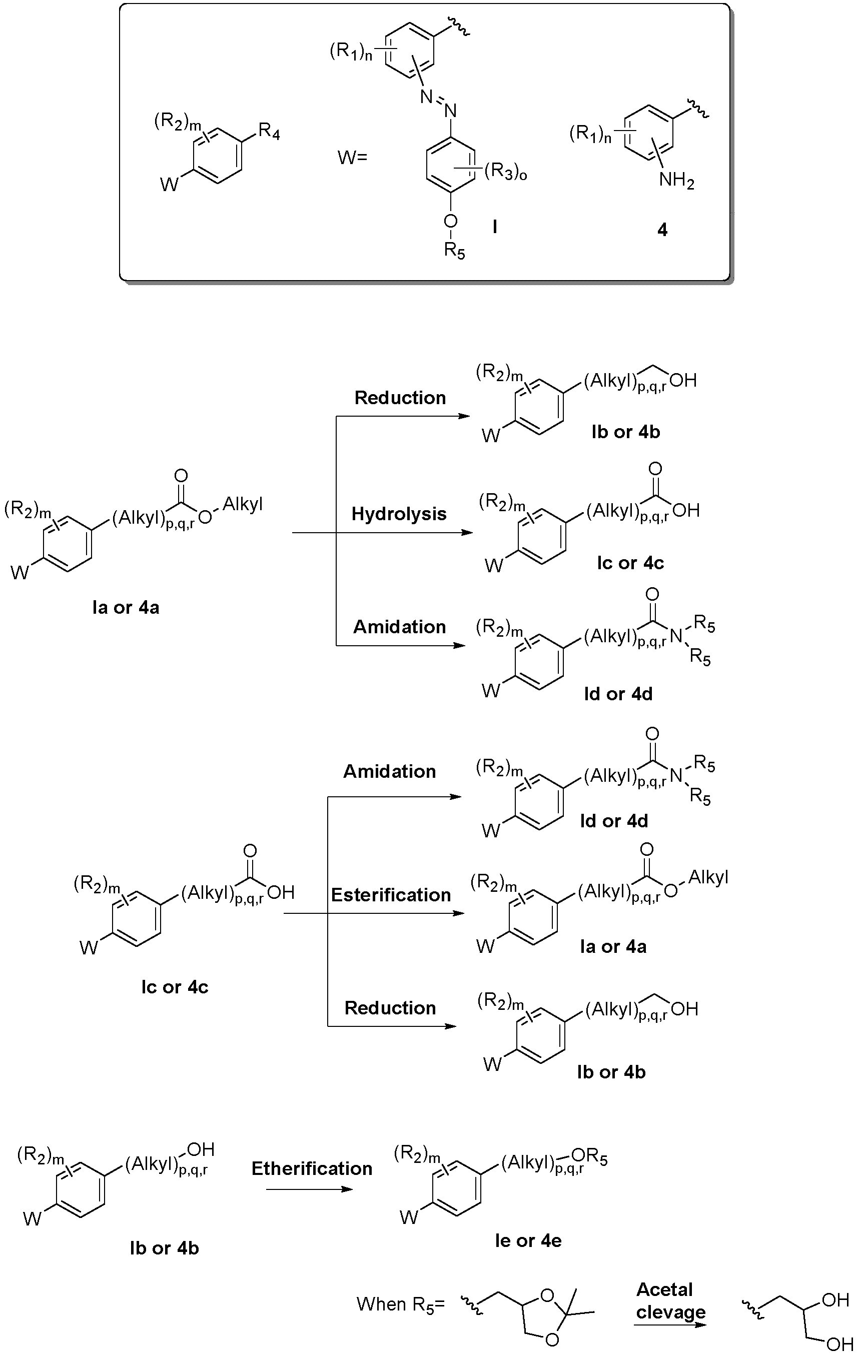

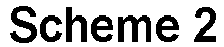

### Abbreviations

- Abs: Absorbance
- ACN: Acetonitrile
- AcOH: Acetic acid
- br: Broad
- CV: Column Volume
- ESI: Electrospray lonisation
- EtOAc: Ethyl Acetate
- EtOH: Ethanol
- FIA: Flux Injected Analysis
- d: Doublet
- dd: Doublet of Doublets
- ddd: Doublet of Doublet of Doublets
- dddd: Doublet of Doublet of Doublet of Doublets
- DAD: Diode Array Detector
- DCM: Dichloromethane
- DIPEA: Diisopropylamine
- DME: Dimethoxiethane
- DMF: *N,N*-Dimethylformamide
- DMSO: Dimethylsulfoxide
- dt: Doublet of triplet
- ee: enantiomeric excess
- ESI: Electrospray Ionization
- h: Hours
- HPLC: High-performance Liquid Chromatography
- IPA: Isopropanol
- LAH: Lithium aluminium hydride
- m: Multiplet
- MeOH: Methanol
- mg: Miligrams
- mL: Mililiter
- µL: Microliter
- MS: Mass Spectroscopy
- MHz: Mega Hertz
- min: Minutes
- mm: Millimetre
- mmol: Milimol
- m/z: Mass to Charge Ratio
- NMR: Nuclear Magnetic Resonance Spectrometry
- nm: Nanometre
- ppm: Parts Per Million
- q: Quartet
- r.t.: room temperature
- RT: Retention Time
- s: Singlet
- t: Triplet
- THF: Tetrahydrofuran
- UV: Ultraviolet

### Materials and methods

All the Chemicals and solvents are from commercial suppliers and used without purification, except the anhydrous solvents such as DMF which were treated previously through a system of solvent purification (PureSolv), degasified with inert gases and dried over alumina or molecular sieves. Reactions were monitored by thin layer chromatography (60 F, 0.2 mm, Macherey-Nagel) by visualisation under 254 and/or 365 nm lamp. Purification was made by Flash column chromatography by using Merck Silica Gel 60, 40-63 microns RE or by Phase Reverse with an Isolera-Biotage equipment (SNAP KP-C18-HS; A: Water/Formic acid (0.05%), B: ACN/Formic Acid (0.05%): 5%B 3CV, 5%B-100%B 18CV, 100%B 5CV). NMR were performed in a Brucker 400 MHz. Chemical shifts δ are reported in parts per million (ppm). Azo compounds existed in two isomeric forms, but in NMR spectra, only was assigned to the trans configuration based on abundance. HPLC 2795 Alliance Waters Aquity coupled to Detector DAD Agilent 1100 and Detector MS Waters ESI triple quadrupole Quattro micro, 10 µL of sample in ACN was injected, using a ZORBAX Extend-C18 3.5 µm 2.1x50mm (Agilent) column. The mobile phase used was a mixture of A = water + 0.05 formic acid and B = ACN + 0.05 formic acid with method described as follows: flow 0.5 mL/min; 5% B for 0.5 min; 5% to 100% B in 5 min, 100% B for 2 min. Mass spectroscopy (MS) analysed by FIA with coupled to LCT Premier Orthogonal Accelerated Time of Flight Mass Spectrometer, acquiring data by ESI in positive mode. Spectra have been scanned between 50 and 1500 Da with values every 0.2 seconds and peaks are given m/z (% of basis peak). The enantioselective analyses and purifications were conducted using standard normal mobile phase comprised of n-hexane in combination with IPA and formic acid or trifluoroacetic acid as additives. UV analyses were performed at fixed wavelength (360 nm) for all compounds. The chiral analytical HPLC system consisted of a Dionex LPG-3400SD, equipped with a Dionex ACC-3000 autosampler, a Dionex VWD-3400-RS UV detector and collection data was performed using Chromeleon software from Thermofisher. Enantiomeric purifications were conducted on a waters 1525P semi-prep HPLC coupled to a waters 2489 UV detector, a waters fraction collector III and collection data was performed using Empower software from Waters. Optical rotations were measured on a Perkin Elmer 341 polarimeter using a Na/Hal lamp set at 589 nm.

### Synthesis of compounds of formula (I)

All compounds were synthesized following one of the general methods:

### General Procedure for the Suzuki-Miyaura Reaction

(Method A). A suspension of arylboronic acid **1, 1'** or **1"** (1.05 eq), aryl bromide **2,2'** or **2"** (1 eq), Na₂CO₃ (1.5 eq) and Pd(Ph₃)₄ (0.05 eq) in 1,4-Dioxane (0.5 M) was heated in a microwave (time 2 h, Potency 300 Watts, Tmax 150°C). Then it was concentrated under reduced pressure, and the residue was purified by silica gel chromatography eluting with hexane: EtOAc (10:1) to provide the bicyclic compound intermediate **3** or **4.**

(Method B). A suspension of arylboronic acid type **1, 1'** or **1"** (1.05 to 1.125 eq), aryl bromide type **2, 2'** or **2"** (1 eq), Na₂CO₃ (1.5 to 4 eq) and Pd(Ph₃)₄ (0.05 eq) in an appropriate solvent (Toluene, H₂O, EtOH or 1,4-Dioxane), (0.2 to 0.5 M) was refluxed overnight. Then was concentrated under reduced pressure, and the residue was purified by silica gel chromatography eluting with hexane: EtOAc (10:1) or by reverse phase to provide the bicyclic intermediate type 3 or 4.

### General Procedure for Nitro Reduction

(Method C). To a solution of nitro derivative type 3 or 9 (1 eq) in MeOH (0.1 M) or MeOH/THF (1:1) (0.05 to 0.015 M) was added Pd-C (10% W/W) and the mixture was stirred for 24 h at r.t. under a hydrogen atmosphere. The reaction mixture was then filtered by using celite, and the filtrate was evaporated under vacuum yielding the amine compound type 4 or 10 respectively, and was used without further purification.

### General Procedure for Ester Reduction

(Method D). To a stirred solution of compounds la, or 4a (1 eq) in anhydrous THF (0.1 M to 0.01M), LAH (4 eq) was added, in small portions, and the reaction mixture was stirred at r.t. for 3h. Then, the excess of LAH was destroyed with sodium sulphate decahydrate, filtered and evaporated to dryness, obtaining the compounds **Ib or 4b.**

### General Procedure for Ether formation (Williamson).

(Method E). To a stirred solution of intermediates type **Ib, 4b** or **8** (1 eq) in appropriate solvent (DMF, EtOH or ACN) (0.1 to 1.5 M), a base (NaH, K₂CO₃, Cs₂CO₃ or NaOH) (1.25 to 2 eq) was added, in small portions or dropwise, and the reaction mixture was stirred at r.t. for 10 min. Then, the alkylating agent (1.1 to 1.3 eq) was added, and the mixture was warmed (60°C to 80°C) overnight. Then, the mixture was treated with EtOAc/water, the organic layer was dried over anhydrous MgSO₄, filtered, and concentrated in vacuum. The residue was purified by flash chromatography (silica gel) to afford compounds **le, 4e** or **9.**

### General Procedure for Nitrosation

(Method F). Amino compound type **4** (1 eq) was dissolved in DCM-Water (1.5-2) (0.1 M) and OXONE (2 eq) were added. The reaction mixture was stirred vigorously at r.t. overnight. The mixture was extracted twice with DCM, the organic layer was dried over anhydrous MgSO₄, filtered and concentrated in vacuum. The residue was purified by flash chromatography (silica gel) to afford pure nitroso compounds type 6.

### General Procedure for Azocompunds formation.

(Method G): Mils reaction A solution of a nitroso compound type 6 (1.1 eq) and an amine type **10** (1 eq) in AcOH [or a mixture of DCM-AcOH (1:2)] (0.1 to 0.01 M), was stirred at rt overnight. Then was concentrated under reduced pressure, and the residue was purified by reverse phase to provide the azocompound.

(Method H): Diazotization To an ice-cold solution of amine type **4** (1eq) in HCl 1M (3 eq) and THF (0.4M) at 0°C was added, dropwise, sodium nitrite (1.2 eq) dissolved in water (0.4M) at 0 °C. The mixture was stirred for 30 min at 0°C and then it was added dropwise (always cold) to an ice-cold solution of phenol type **5** (1.2 eq) in aqueous NaOH 1M (3 eq). the reaction mixture was kept and stirred at 0-5 °C. for 1h. The residue was taken in EtOAc and water (add HCl 1M until acid pH), extracted twice with EtOAc. Combined organic layers were washed with Brine solution, dried over MgSO₄, filtered and evaporated under reduced pressure. The solid was digested with DCM and filter, yielding the azophenol type **7.**

### General Procedure for separation of enantiomer from racemic samples and enantiomeric excess analysis of chiral compounds.

Method J1. Enantiomeric separation of racemic sample was accomplished using a Chiralpak IB column (10x250mm, 5µm), and an isocratic mixture of hexane and IPA at 97:3, containing a 0.1% of formic acid. Flow was set at 5ml/min for 30 min and fractions containing pure enantiomer were evaporated together to afford each enantiomer separately. Enantiomeric excess was determined using a Chiralpak IB column (4.6x250mm, 5µm), and an isocratic mixture of hexane and IPA at 97:3, containing a 0.1 % of formic acid at 1ml/min.

Method J2. Enantiomeric excess was determined using a Lux Amylose-2 column (4.6x250mm, 5µm) and a mixture of hexane-EtOH in an 85:15 ratio at a flow rate of 1ml/min.

Method J3. Enantiomeric separation of racemic sample was accomplished using a Chiralpak IB column (10x250mm, 5µm), and an isocratic mixture of hexane and IPA at 9:1, containing a 0.1% of formic acid. Flow was set at 5ml/min for 30 min and fractions containing pure enantiomer were evaporated together to afford each enantiomer separately. Enantiomeric excess was determined using a Chiralpak IB column (4.6x250mm, 5µm), and an isocratic mixture of hexane and IPA at 9:1, containing a 0.1 % of formic acid at 1ml/min.

Method J4. Enantiomeric excess was determined using a Lux Amylose-2 column (4.6x250mm, 5µm) and a mixture of hexane-EtOH in an 85:15 ratio containing a 0.1% of trifluoroacetic acid at a flow rate of 1ml/min.

Method J5. Enantiomeric separation of racemic sample was accomplished using a Chiralpak IB column (10x250mm, 5µm), and an isocratic mixture of hexane and IPA at 9:1. Flow was set at 5ml/min for 30 min and fractions containing pure enantiomer were evaporated together to afford each enantiomer separately. Enantiomeric excess was determined using a Chiralpak IB column (4.6x250mm, 5µm), and an isocratic mixture of hexane and IPA at 9:1 at 1ml/min.

### General Procedure for Hydrolysis

### (Method K). To a solution of ester compound la (1 eq) in THF or THF-MeOH (3:1)

(0.05 M) was added a solution of LiOH 1M (10 eq). The mixture was stirred at r.t. for overnight, then was acidified with HCl 1N to pH~4-5. Finally, it was concentrated in vacuo and purified by Reverse Phase yielding the acid compound Ic.

### General Procedure for amidation.

(Method M). DIPEA (2 eq) was added to a stirred solution of acid Ic (1 eq) in DCM (0.01 M). 1-Hydroxybenzotriazole hydrate (1.5 eq) and 1-etil-3-(3-dimetilaminopropil)carbodiimide clorhydrate (1.5 eq) were then added at room temperature. After 30 minutes, appropriate commercial amine (1.1 eq) was added and the resulting mixture was stirred at room temperature overnight. Then, the mixture was concentrated under reduced pressure and purified by reverse phase to obtain the amide compound Id.

### General Procedure for acetal cleavage

(Method N). To a solution of acetal le (1 eq) in MeOH (2.5 M) a solution of HCl 4M in dioxane (30 eq) was added. The mixture was stirred at r.t. for 2 h, then was concentrated in vacuo yielding the deprotected compound.

### General Procedure for Ester formation

(Method R). To a stirred solution of acid Ic (1 eq) in anhydrous MeOH (0.1 M), thionyl chloride (20 eq) was added, dropwise, and the reaction mixture was stirred at r.t. for 2h. Then, the solution was evaporated to dryness, obtaining ester la.

### Intermediates

### Methyl 2-methyl-3'-nitro-[1,1'-biphenyl]-4-carboxylate (3-1)

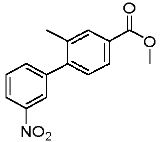

According to method B, from 1.31g of methyl 4-bromo-3-methylbenzoate, 1g of (3-nitrophenyl)boronic acid and in dioxane (0.5 M), 533 mg of (3-1) were obtained (34% yield) as a white solid.

¹H-NMR (400 MHz, CDCl₃) δ 8.23 (dt, *J* = 7.6, 1.5 Hz, 1H), 8.18 (t, *J* = 1.5 Hz, 1H), 7.97 (s,1H), 7.91 (d, *J* = 8 Hz,1H), 7.65 (dt, *J* = 7.6, 1.5 Hz, 1H), 7.60 (t, *J* = 7.6 Hz, 1H), 7.28 (d, *J* = 8 Hz, 1H), 3.93 (s, 3H), 2.30 (s, 3H).

¹³C NMR (101 MHz, CDCl₃) δ 166.9, 148.3, 143.9, 142.6, 135.7, 135.1, 131.9, 130.1, 129.9, 129.4, 127.4, 124.0, 122.5, 52.4, 20.4.

### Ethyl 4'-nitro-[1,1'-biphenyl]-4-carboxylate (3-2)

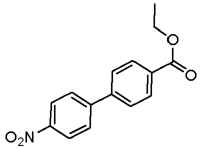

According to method B, from 300 mg of 1-bromo-4-nitrobenzene, 324 mg of (4-(ethoxycarbonyl)phenyl)boronic acid and 394 mg of Na₂CO₃, in toluene/EtOH/H₂O (0.2 M), 360 mg of (3-2) were obtained (90% yield).

¹H-NMR (400 MHz, CDCl₃) δ 8.31 (d, *J* = 8.8 Hz, 2H), 8.16 (d, *J* = 8.4 Hz, 2H), 7.77 (d, *J* = 8.8 Hz, 2H), 7.69 (d, *J = 8.4* Hz, 2H), 4.42 (q, *J* = 7.1 Hz, 2H), 1.43 (t, *J = 7.1* Hz, 3H).

### Ethyl 3'-methyl-4'-nitro-[1,1'-biphenyl]-4-carboxylate (3-3)

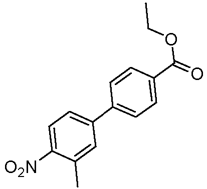

According to method B, from 300 mg of 4-bromo-2-methyl-1-nitrobenzene, 333 mg of (4-(ethoxycarbonyl)phenyl)boronic acid and 368 mg of Na₂CO₃, in toluene/EtOH/H₂O (0.2 M), 333 mg of (3-3) were obtained (84% yield).

¹H-NMR (400 MHz, CDCl₃) δ 8.06 (d, *J* = 8.3 Hz, 2H), 8.00 (d, *J* = 9.0 Hz, 1H), 7.58 (d, *J* = 8.3 Hz, 2H), 7.50-7.46 (m, 2H), 4.33 (q, *J* = 7.1 Hz, 2H), 2.61 (s, 3H), 1.34 (t, *J* = 7.1 Hz, 3H).

### 2-(3'- Methyl-4'-nitro-[1,1'-biphenyl]-4-yl)acetic acid (3-4)

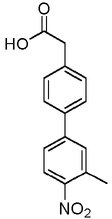

According to method B, from 300 mg of 4-bromo-2-methyl-1-nitrobenzene, 281 mg of 2-(4-boronophenyl)acetic acid in Toluene/EtOH/water (0.2 M), 226 mg of (3-4) were obtained (60% yield) once it was purified by reverse phase.

¹H NMR (400 MHz, CDCl₃) δ 8.10 (d, *J =* 9.0 Hz, 1H), 7.60 (d, *J =* 8.3 Hz, 2H), 7.56-7.51 (m, 2H), 7.43 (d, *J* = 8.3 Hz, 2H), 3.75 (s, 2H), 2.71 (s, 3H).

### Methyl 3'-amino-2-methyl-[1,1'-biphenyl]-4-carboxylate (4a-1)

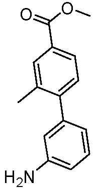

According to method C, from 250 mg of compound (3-1) in MeOH, 220 mg of (4a-1) (99% yield) were obtained.

¹H-NMR (400 MHz, CDCl₃) δ 7.91 (s, 1H), 7.85 (dd, *J* = 8.0, 2 Hz, 1H), 7.26 (d, *J =* 8.0 Hz, 1H), 7.19 (t, *J* = 8.0 Hz, 1H), 6.68 (dd, *J* = 8.0, 2.0 Hz, 2H), 6.60 (t, *J* = 2 Hz, 1H), 3.91 (s, 3H), 3.71 (brs, 2H), 2.30 (s, 3H).

¹³C-NMR (101 MHz, CDCl₃) δ 167.37, 146.98, 146.35, 142.28, 135.81, 131.52, 129.85, 129.27, 128.91, 126.99, 119.46, 115.69, 114.22, 52.21, 20.56.

### Ethyl 4'-amino-[1,1'-biphenyl]-4-carboxylate (4a-2)

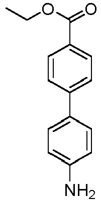

According to method B, from 300 mg of 4-bromoaniline, 381 mg of ((4-(ethoxycarbonyl)phenyl)boronic acid and 462 mg of Na₂CO₃, in toluene/EtOH/H₂O (0.2 M), 102 mg of **(4a-2)** were obtained (24% yield).

According to method C, from 24 mg of compound **(3-2)** in MeOH-THF (0.015 M), 21 mg of **(4a-2)** (88% yield) were obtained.

¹H-NMR (400 MHz, CDCl₃) δ 8.09 (d, *J* = 8.5 Hz, 2H), 7.62 (d, *J* = 8.5 Hz, 2H), 7.49 (d, *J* = 8.5 Hz, 2H), 6.80 (d, *J* = 8.5 Hz, 2H), 4.42 (q, *J* = 7.2 Hz, 2H), 3.86 (brs, 2H), 1.43 (t, *J* = 7.2 Hz, 3H).

### Methyl 4'-amino-2-methyl-[1,1'-biphenyl]-4-carboxylate (4a-3)

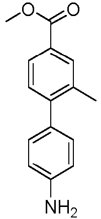

According to method B, from 200 mg of methyl 4-bromo-3-methylbenzoate, 381 mg of (4-aminophenyl)boronic acid hydrochloride, and 231 mg of Na₂CO₃, in 1,4-dioxane (0.2 M), 117 mg of **(4a-3)** were obtained (56% yield).

¹H-NMR (400 MHz, CDCl₃) δ 7.84 (d, *J* = 1.9 Hz, 1H), 7.78 (dd, *J* = 8.0, 1.9 Hz, 1H), 7.18 (d, *J* = 8.0 Hz, 1H), 7.04 (d, *J* = 8.5 Hz, 2H), 6.65 (d, *J* = 8.5 Hz, 2H), 3.83 (s, 3H), 2.24 (s, 3H).

### Ethyl 4'-amino-3'-methyl-[1,1'-biphenyl]-4-carboxylate (4a-4)

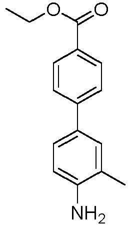

According to method C, from 304 mg of compound (3-3) in MeOH-THF (0.05 M), 271 mg of (4a-4) (99% yield) were obtained.

¹H-NMR (400 MHz, CDCl₃) δ 7.97 (d, *J* = 8.6 Hz, 2H), 7.51 (d, *J* = 8.6 Hz, 2H), 7.30-7.22 (m, 2H), 6.66 (d, *J* = 8.0 Hz, 1H), 4.30 (q, *J* = 7.1 Hz, 2H), 3.73-3.60 (brs, 2H), 2.15 (s, 3H), 1.32 (t, *J* = 7.1 Hz, 3H).

### 3'-Amino-[1,1'-biphenyl]-4-yl)methanol (4b-1)

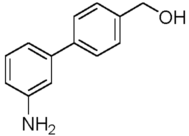

According to method D, from 200 mg of commercial methyl 3'-amino-[1,1'-biphenyl]-4-carboxylate in THF (0.1 M), 174 mg of (4b-1) (99% yield) were obtained.

¹H-NMR (400 MHz, CDCl₃) δ 7.48 (d, *J* = 8.2 Hz, 2H), 7.34 (d, *J* = 8.2 Hz, 2H), 7.15 (t, J = 7.8 Hz, 1H), 6.91 (ddd, *J* = 7.8, 2.0, 1.0 Hz, 1H), 6.83 (t, *J* = 2.0 Hz, 1H), 6.61 (ddd, *J* = 7.8, 2.0, 1.0 Hz, 1H), 4.65 (s, 2H).

### (3'-Amino-2-methyl-[1,1'-biphenyl]-4-yl)metanol (4b-2)

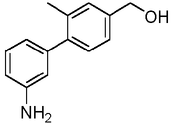

According to method D, from 75 mg of compound **(4a-1)** in THF (0.1 M), 38 mg of **(4b-2)** (57% yield) were obtained.

¹H-NMR (400 MHz, CDCl₃) δ 7.20-7.08 (m, 4H), 6.62 (dt, *J* = 7.8, 1.3 Hz, 1H), 6.59 (ddd, *J* = 7.8, 2.4, 1.2 Hz, 1H), 6.55-6.52 (m, 1H), 4.61 (s, 2H), 2.20 (s, 3H).

### (+/-)-4'-(((2,2-Dimethyl-1,3-dioxolan-4-yl)methoxy)methyl)-[1,1'-biphenyl]-3-amine ((+/-)-4e-1)

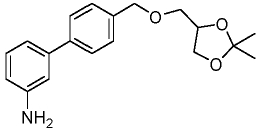

According to method E, from 175 mg of **(4b-1),** 277mg of (2,2-dimethyl-1,3-dioxolan-4-yl)methyl 4-methylbenzenesulfonate and 52 mg of NaH (60%) in DMF (0.1 M), 62 mg of **((+/-)-4e-1)** were obtained (23% yield) once it was purified by using (hexane-EtOAc, 1:2) as solvent.

¹H-NMR (400 MHz, CDCl₃) δ 7.57 (d, *J* = 8.2 Hz, 2H), 7.40 (d, *J* = 8.2 Hz, 2H), 7.25 (t, *J =* 7.8 Hz, 1H), 7.01 (ddd, *J* = 7.8, 2, 1.0 Hz, 1H), 6.92 (t, *J* = 2.0 Hz, 1H), 6.70 (ddd, *J* = 7.8, 2.0, 1.0 Hz, 1H), 4.66 (d, *J* = 12.0 Hz, 1H), 4.61 (d, *J* = 12.0 Hz, 1H), 4.35 (ddd, *J* = 12.0, 6.4, 5.6 Hz, 1H), 4.10 (dd, *J* = 8.2, 6.4 Hz, 1H), 3.79 (dd, *J* = 8.2, 6.4 Hz, 2H), 3.61 (dd, *J* = 9.8, 5.6 Hz, 1H), 3.53 (dd, *J* = 9.8, 5.6 Hz, 1H), 1.46 (s, 3H), 1.40 (s, 3H).

### 2-(4'-Amino-3'-methyl-[1,1'-biphenyl]-4-yl)acetic acid (4c-1)

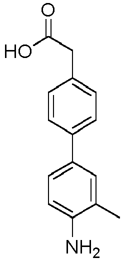

According to method C, from 225 mg of compound **(3-4)** in MeOH/THF (0.03 M), 192 mg of (4c-1) (96% yield) were obtained.

¹H NMR (400 MHz, CD₃OD) δ 7.49 (d, *J* = 8.2 Hz, 2H), 7.29 (d, *J* =2.0 Hz, 1H), 7.29 (d, *J* =8.2 Hz, 1H), 7.26 (dd, *J* = 8.2, 2.0 Hz, 1H), 6.80 (d, *J* = 8.2 Hz, 1H), 3.61 (s, 2H), 2.23 (s, 3H).

### Methyl 3'-nitroso-[1,1'-biphenyl]-4-carboxylate (6-1)

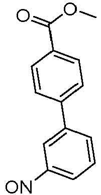

According to method F, from 2.53 g of commercial methyl 3'-amino-[1,1'-biphenyl]-4-carboxylate, 869 mg of **(6-1)** were obtained (32% yield).

¹H NMR (400 MHz, DMSO-δ₆) δ 8.16 (d, *J* = 8.4 Hz, 2H), 8.13 (t, *J =* 1.9 Hz, 1H), 7.98 (ddd, *J* = 7.8, 1.9, 1.1 Hz, 1H), 7.94 (ddd, *J* = 7.8, 1.9, 1.1 Hz, 1H), 7.73 (d, *J* = 8.4 Hz, 2H), 7.73 (t, *J* = 7.8 Hz, 1H), 3.96 (s, 3H).

### Methyl 2-methyl-3'-nitroso-[1,1'-biphenyl]-4-carboxylate (6-2)

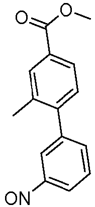

According to method F, from 322 mg of **(4a-1),** 72 mg of **(6-2)** were obtained (21% yield).

¹H-NMR (400 MHz, CDCl₃) δ 8.01 (t, *J* = 1.8 Hz, 1H), 7.97-7.92 (m, 2H), 7.85 (t, *J* = 1.2 Hz, 1H), 7.73-7.66 (m, 2H), 7.34 (d, *J* = 7.9 Hz, 1H), 3.95 (s, 3H), 2.34 (s, 3H).

### Ethyl 4'-nitroso-[1,1'-biphenyl]-4-carboxylate (6-3)

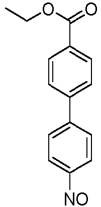

According to method F, from 102 mg of **(4a-2),** 94 mg of **(6-3)** were obtained (87% yield).

¹H-NMR (400 MHz, CDCl₃) δ 7.99 (d, *J* = 8.5 Hz, 2H), 7.52 (d, *J* = 8.5 Hz, 2H), 7.40 (d, *J* = 8.5 Hz, 2H), 6.72 (d, *J* = 8.5 Hz, 2H), 4.33 (q, *J* = 7.2 Hz, 2H), 1.34 (t, *J = 7.2* Hz, 3H).

### Methyl 2-methyl-4'-nitroso-[1,1'-biphenyl]-4-carboxylate (6-4)

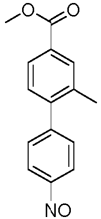

According to method F, from 117 mg of (4a-3), 12 mg of (6-4) were obtained (10% yield).

HPLC-MS: retention time= 3.41 min; m/z: 242 [M+1]⁺.

### Ethyl 3'-methyl-4'-nitroso-[1,1'-biphenyl]-4-carboxylate (6-5)

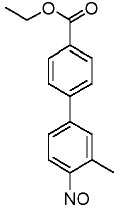

According to method F, from 230 mg of (4a-4), 49 mg of (6-5) was obtained (20% yield).

¹H-NMR (400 MHz, CDCl₃) δ 8.14 (d, *J* = 8.5 Hz, 2H), 7.78 (d, *J* = 2.0 Hz, 1H), 7.70 (d, *J* = 8.5 Hz, 2H), 7.40 (dd, *J* = 8.4, 2.0 Hz, 1H), 6.38 (d, *J* = 8.4 Hz, 1H), 4.42 (q, *J* = 7.1 Hz, 2H), 3.42 (s, 3H), 1.42 (t, *J* = 7.1 Hz, 3H).

### (+/-)-2,2-Dimethyl-4-(((3'-nitroso-[1,1'-biphenyl]-4-yl)methoxy)methyl)-1,3-dioxolane ((+/-)-6-6)

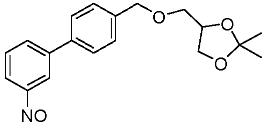

According to method F, from 51 mg of **(+/-)-4e-1,** 8 mg of **((+/-)-6-6)** were obtained (15% yield).

¹H NMR (400 MHz, CDCl₃) δ 8.10 (t, *J* = 1.9 Hz, 1H), 7.94 (ddd, *J* = 7.8, 1.9, 1.2 Hz, 1H), 7.88 (ddd, *J* = 7.8, 1.9, 1.2 Hz, 1H), 7.68 (t, *J* = 7.8 Hz, 1H), 7.64 (d, *J* = 8.2 Hz, 2H), 7.47 (d, *J* = 8.2 Hz, 2H), 4.66 (d, *J* = 12.3 Hz, 1H), 4.62 (d, *J* = 12.3 Hz, 1H), 4.33 (tt, *J* = 6.4, 5.6 Hz, 2H), 4.08 (dd, *J* = 8.3, 6.4 Hz, 1H), 3.77 (dd, *J* = 8.3, 6.4 Hz, 1H), 3.60 (dd, *J* = 9.8, 5.6 Hz, 1H), 3.53 (dd, *J* = 9.8, 5.6 Hz, 1H), 1.43 (s, 3H), 1.37 (s, 3H).

### 2-(4'-((4-Hydroxy-2-methylphenyl)diazenyl)-3'-methyl-[1,1'-biphenyl]-4-yl)acetic acid (7-1)

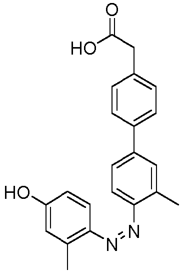

According to method H, from 75 mg of compound **(4c-1)** and 40 mg of m-Cresol, 62 mg of (7-1) (55% yield) were obtained.

¹H NMR (400 MHz, CD₃OD) δ 7.67 (d, *J* = 8.0 Hz, 2H), 7.64 (d, *J* = 8.8 Hz, 1H), 7.63 (d, *J* = 8.8 Hz, 1H), 7.63 (d, *J = 1.2* Hz, 1H), 7.52 (dd, *J* = 8.5, 2.1 Hz, 1H), 7.39 (d, *J* = 8.0 Hz, 2H), 6.77 (d, *J* = 2.8 Hz, 1H), 6.69 (dd, *J* = 8.8, 2.8 Hz, 1H), 3.66 (s, 2H), 2.76 (s, 3H), 2.69 (s, 3H).

### 2-(4'-((4-Hydroxy-3-methylphenyl)diazenyl)-3'-methyl-[1,1'-biphenyl]-4-yl)acetic acid (7-2)

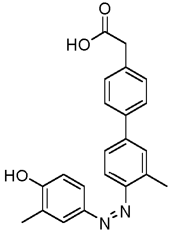

According to method H, from 75 mg of compound **(4c-1)** and 40 mg of o-Cresol, 66 mg of **(7-2)** (59% yield) were obtained.

¹H NMR (400 MHz, CD₃OD) δ 7.72 (d, *J* = 2.0 Hz, 1H), 7.66 (dd, *J* = 8.0. 2.0 Hz, 1H), 7.65 (d, *J* = 8.0 Hz, 2H), 7.65 (d, *J* = 8.0 Hz, 1H), 7.62 (d, *J* = 2.0 Hz, 1H), 7.52 (dd, *J* = 8.4, 2.0 Hz, 1H), 7.39 (d, *J* = 8.0 Hz, 2H), 6.88 (d, *J* = 8.4 Hz, 1H), 3.66 (s, 2H), 2.75 (s, 3H), 2.28 (s, 3H).

### (+/-)-3,3-Dimethyl-1-(3-methyl-4-nitrophenoxy)butan-2-ol ((+/-)-9-1)

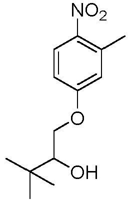

According to method E, from 560 mg of 3-methyl-4-nitrophenol, 490 µL of *2-(tert-*butyl)oxirane and 371 mg of K₂CO₃, 12 mL of DMF, 770 mg of **((+/-)-9-1)** were obtained (39% yield) once it was purified by using (Hexane-EtOAc, 7:3) as solvent.

¹H NMR (400 MHz, CDCl₃) δ 8.06 (d, *J* = 8.7 Hz, 1H), 6.79 (dd, *J* = 8.7, 2.8 Hz, 1H), 6.78 (d, *J = 2.8* Hz, 1H), 4.15 (dd, *J* = 9, 2.4 Hz, 1H), 3.92 (t, *J =* 9 Hz, 1H), 3.68 (dt, *J* = 9, 2.4 Hz, 1H), 2.61 (s, 3H), 1.00 (s, 9H)

### (-)-(R)-3,3-dimethyl-1-(3-methyl-4-nitrophenoxy)butan-2-ol ((-)-9-1)

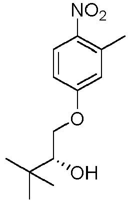

According to method E, from 2.89 g of 3-methyl-4-nitrophenol, 6.17g of (*R*)-2-hydroxy-3,3-dimethylbutyl 4-methylbenzenesulfonate (prepared as is described in US2005/0043368 for the S-enantiomer) and 5.22 g of K₂CO₃, 63 mL of DMF, 172 mg of **((-)-9-1)** were obtained (4% yield) once it was purified by using (Hexane-EtOAc, 7:3) as solvent.
[α] D₂₀ = -20 (c 1.1, CHCl₃)

### (+)-(S)-3,3-dimethyl-1-(3-methyl-4-nitrophenoxy)butan-2-ol ((+)-9-1)

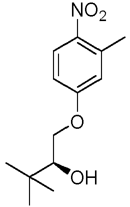

According to method E, from 2.04 g of 3-methyl-4-nitrophenol, 5.43 g of (S)-2-hydroxy-3,3-dimethylbutyl 4-methylbenzenesulfonate (prepared as is described in US2005/0043368) and 3.67 g of K₂CO₃, 44 mL of DMF, 550 mg of ((+)-9-1) were obtained (16% yield) once it was purified by using (Hexane-EtOAc, 7:3) as solvent.
[α] D₂₀ = +28 (c 0.3, CHCl₃)

### (+/-)-3,3-Dimethyl-1-(2-methyl-4-nitrophenoxy)butan-2-ol ((+/-)-9-2)

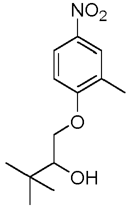

According to method E, from 450 mg of 2-methyl-4-nitrophenol, 290 µL of 2*-(tert-*butyl)oxirane and 370 mg of potassium carbonate in 10 mL of DMF, 360 mg of ((+/-)-**9-2)** were obtained (48% yield) once it was purified by using (hexane-EtOAc, 3:1) as solvent.

¹H NMR (400 MHz, CDCl₃) δ 8.06 (d, *J* = 9 Hz, 1H), 8.01 (s, 1H), 6.85 (d, *J* = 9.0 Hz, 1H), 4.20 (dd, *J* = 9.2, 2.6 Hz, 1H), 3.99 (t, *J* = 9.2 Hz, 1H), 3.75 (dd, *J* = 9.2, 2.6 Hz, 1H), 2.27 (s, 3H), 1.02 (s, 9H).

### (+/-)-1-(4-Amino-3-methylphenoxy)-3,3-dimethylbutan-1-ol ((+/-)-10-1)

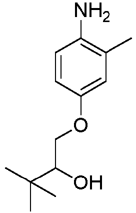

According to method C, from 770 mg of compound **((+/-)-9-1)** in MeOH (0.3 M), 635 mg of **((+/-)-10-1)** (94% yield) were obtained.

¹H NMR (400 MHz, CDCl₃) δ 6.88 (d, *J* = 8.6 Hz, 1H), 6.71 (d, *J* = 2.9 Hz, 1H), 6.66 (dd, *J* = 8.6, 2.9 Hz, 1H), 4.03 (dd, *J* = 9.1, 2.5 Hz, 1H), 3.78 (t, *J* = 9.1 Hz, 1H), 3.63 (dd, *J* = 9.1, 2.5 Hz, 1H), 2.26 (s, 3H), 0.98 (s, 9H).

### (-)-(R)-1-(4-Amino-3-methylphenoxy)-3,3-dimethylbutan-2-ol ((-)-10-1)

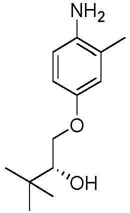

According to method C, from 172 mg of compound **((-)-9-1)** in MeOH (0.05 M), 150 mg of **((-)-10-1)** (99% yield) were obtained.

¹H NMR (400 MHz, CD₃OD) δ 6.69 (d, *J* = 8.6 Hz, 1H), 6.67 *(d, J* = 2.8 Hz, 1H), 6.62 (dd, *J* = 8.6, 2.8 Hz, 1H), 4.03 (dd, *J* = 9.9, 2.7 Hz, 1H), 3.77 (dd, *J* = 9.9, 8.2 Hz, 1H), 3.55 (dd, J= 8.2, 2.7 Hz, 1H), 2.15 (s, 3H), 0.97 (s, 9H).
[α] D₂₀ = -32 (c 0.07, MeOH).

### (+)-(S)-1-(4-Amino-3-methylphenoxy)-3,3-dimethylbutan-2-ol ((+)-10-1)

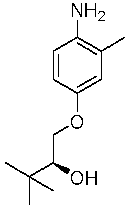

According to method C, from 550 mg of compound ((+)-9-1) in MeOH (0.05 M), 450 mg of **((+)-10-1)** (93% yield) were obtained.

¹H NMR (400 MHz, CD₃OD) δ 6.69 (d, *J* = 8.6 Hz, 1H), 6.67 (d, *J* = 2.8 Hz, 1H), 6.62 (dd, J = 8.6, 2.8 Hz, 1H), 4.04 (dd, *J* = 9.9, 2.7 Hz, 1H), 3.77 (dd, *J* = 9.9, 8.2 Hz, 1H), 3.54 (dd, *J* = 8.2, 2.7 Hz, 1H), 2.16 (s, 3H), 0.98 (s, 9H).
[α] D₂₀ = +37 (c 0.2, MeOH)

### (+/-)-1-(4-Amino-2-methylphenoxy)-3,3-dimethylbutan-2-ol ((+/-)-10-2)

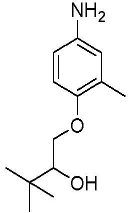

According to method C, from 360 mg of compound **((+/-)-9-2)** in MeOH (0.05 M), 317 mg of **((+/-)-10-2)** (100% yield) were obtained.

¹H NMR (400 MHz, CDCl₃) δ 6.67 (d, *J* = 8.5 Hz, 1H), 6.57 (d, *J* = 2.8 Hz, 1H), 6.52 (dd, *J* = 8.5, 2.8 Hz, 1H), 4.02 (dd, *J* = 9.0, 2.6 Hz, 1H), 3.79 (t, *J* = 9.0 Hz, 1H), 3.68 (dd, *J* = 9.0, 2.6 Hz, 1H), 2.18 (s, 3H), 1.00 (s, 9H).

¹³C NMR (101 MHz, CDCl₃) δ 150.1, 140.0, 128.0, 118.61, 113.5, 113.3, 77.4, 70.5, 33.6, 26.1, 16.4.

### EXAMPLES

### EXAMPLE 1 (+/-)-Methyl 3'-((4-(2-hydroxy-3,3-dimethylbutoxy)-2-methylphenyl)diazenyl)-[1,1'-biphenyl]-4-carboxylate ((+/-)-Ia-1)

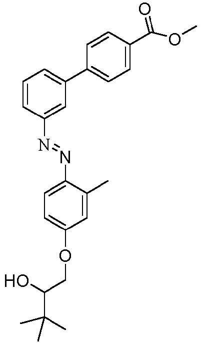

According to method G, from 112 mg of nitroso (6-1) and 94 mg of amine ((+/-)-10-1) in AcOH:DCM (0.04 M), 92 mg of ((+/-)-Ia-1) (49% yield) were obtained.

¹H NMR (400 MHz, CDCl₃) δ 8.14 (d, *J* = 8.5 Hz, 2H), 8.14 (t, *J* = 1.9 Hz, 1H), 7.90 (ddd, *J* = 7.8, 1.9, 1.1 Hz, 1H), 7.75 (d, *J* = 8.5 Hz, 2H), 7.74 (d, *J* = 9.2 Hz, 1H), 7.69 (ddd, *J* = 7.8, 1.9, 1.1 Hz, 1H), 7.58 (t, *J* = 7.8 Hz, 1H), 6.87 (d, *J* = 2.8 Hz, 1H), 6.82 (dd, J= 8.9, 2.8 Hz, 1H), 4.18 (dd, *J* = 9.2, 2.4 Hz, 1H), 3.95 (s, 3H), (t, *J* = 9.2 Hz, 4H), 3.72 (dd, *J* = 9.2, 2.4 Hz, 1H), 2.75 (s, 3H), 1.04 (s, 9H).

### EXAMPLE 2 (-)-(R)-Methyl 3'-((4-(2-hydroxy-3,3-dimethylbutoxy)-2-methylphenyl)diazenyl)-[1,1'-biphenyl]-4-carboxylate ((-)-Ia-1)

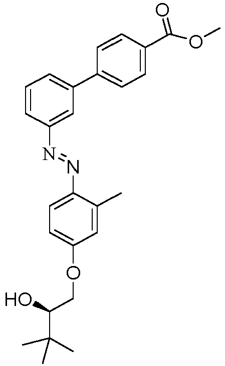

According to method G and J2, from 62 mg of nitroso **(6-1)** and 52 mg of amine **((-)-10-1)** in AcOH:DCM (0.04 M), 64 mg of **((-)-la-1)** (62% yield) were obtained in a 89% ee

According to method J1, from 6.8 mg of racemic **((+/-)-Ia-1),** 1.1 mg of ((-)-la-1) (32% yield) were obtained in a 100% ee.
[α] D₂₀ = -12 (c 0.1, CHCl₃)

### EXAMPLE 3 (+)-(S)-Methyl 3'-((4-(2-hydroxy-3,3-dimethylbutoxy)-2-methylphenyl)diazenyl)-[1,1'-biphenyl]-4-carboxylate ((+)-Ia-1)

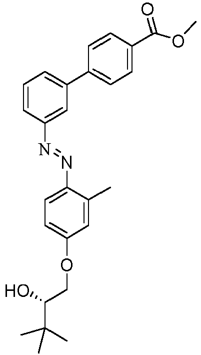

According to method G and J2, from 59 mg of nitroso (6-1) and 50 mg of amine ((+)-**10-1)** in AcOH:DCM (0.04 M), 57 mg of ((+)-la-1) (57% yield) were obtained in a 89% ee.

According to method J1, from 6.8 mg of racemic **((+/-)-la-1),1.1** mg of **((+)-la-1)** (32% yield) were obtained in a 98% ee.
[α] D₂₀ = +8 (c 0.1, CHCl₃)

### EXAMPLE 4 (+/-)-1-(4-((4'-(Hydroxymethyl)-[1,1'-biphenyl]-3-yl)diazenyl)-2-methylphenoxy)-3,3-dimethylbutan-2-ol ((+/-)-Ia-2)

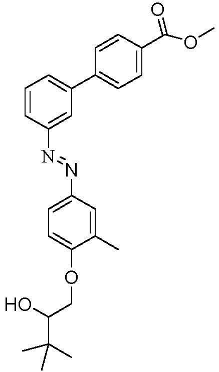

According to method G, from 31 mg of nitroso (6-1) and 26 mg of amine ((+/-)-10-2) in AcOH:DCM (0.04 M), 57 mg of ((+/-)-Ia-2) (56% yield) were obtained.

¹H NMR (400 MHz, CDCl₃) δ 8.02 (d, *J =* 8.5 Hz, 2H), 8.02 (t, *J =* 1.9 Hz, 1H), 7.78 (ddd, *J* = 7.9, 2.0, 1.1 Hz, 1H), 7.71 (dd, *J* = 8.4, 2.4 Hz, 1H), 7.69 (d, *J* = 2.4 Hz, 1H), 7.64 (d, *J* = 8.5 Hz, 2H), 7.58 (ddd, *J* = 7.8, 1.9, 1.1 Hz, 1H), 7.47 (t, *J* = 7.8 Hz, 1H), 6.84 (d, *J* = 8.4 Hz, 1H), 4.10 (dd, *J* = 9.2, 2.6 Hz, 1H), 3.87 (t, *J =* 9.2 Hz, 1H), 3.83 (s, 3H), 3.65 (dt, *J* = 9.2, 2.6 Hz 1H), 2.21 (s, 3H), 0.93 (s, 9H).

### EXAMPLE 5 (+/-)-Methyl 3'-((4-(2-hydroxy-3,3-dimethylbutoxy)-2-methylphenyl)diazenyl)-2-methyl-[1,1'-biphenyl]-4-carboxylate ((+/-)-Ia-3)

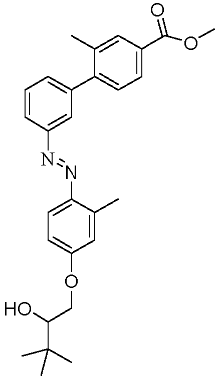

According to method G, from 72 mg of nitroso **(6-2)** and 57 mg of amine **((+/-)-10-1)** in AcOH:DCM (0.04 M), 39 mg of **((+/-)-Ia-3)** (33% yield) were obtained.

¹H NMR (400 MHz, CDCl₃) δ 7.99 (s, 1H), 7.93 (dd, *J* = 8.0, 1.2 Hz, 1H), 7.89 (ddd, *J* = 8.0, 1.6, 1.2 Hz, 1H), 7.84 (t, *J* = 1.6 Hz, 1H), 7.73 (d, *J* = 9.0 Hz, 1H), 7.56 (t, *J* = 8.0 Hz, 1H), 7.39 (dt, *J* = 8.0, 1.6 Hz, 2H), 7.38 (d, *J* = 8.0, 1H), 6.85 (d, *J* = 2.8 Hz, 1H), 6.81 (dd, *J* = 9.0, 2.8 Hz, 1H), 4.17 (dd, *J* = 9.0, 2.8 Hz, 1H), 3.95 (s, 3H), 3.94 (t, *J* = 9.0 Hz, 1H), 3.72 (dd, *J* = 9.0, 2.4 Hz, 1H), 2.72 (s, 3H), 2.37 (s, 3H), 1.04 (s, 9H).

### EXAMPLE 6 (+/-)-Methyl 3'-((4-(2-hydroxy-3,3-dimethylbutoxy)-3-methylphenyl)diazenyl)-2-methyl-[1,1'-biphenyl]-4-carboxylate ((+/-)-Ia-4)

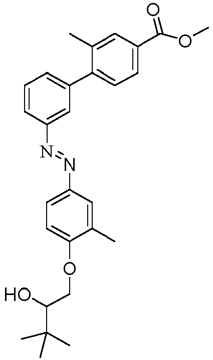

According to method G, from 62 mg of nitroso **(6-2)** and 49 mg of amine **((+/-)-10-2)** in AcOH:DCM (0.04 M), 37 mg of **((+/-)-Ia-4)** (33% yield) were obtained.

¹H NMR (400 MHz, CDCl₃) δ 7.98 (d, *J =* 1.8 Hz, 1H), 7.92 (dd, J = 8.0, 1.2 Hz, 1H), 7.89 (ddd, *J* = 8.0, 1.8, 1.2 Hz, 1H), 7.84 (t, *J* = 1.8 Hz, 1H), 7.81 (dd, *J* = 8.4, 2.8 Hz, 1H), 7.79 (d, *J =* 2.8 Hz, 1H), 7.56 (t, *J =* 8.0 Hz, 1H), 7.39 (dt, *J* = 8.0, 1.2 Hz, 1H), 7.37 (d, *J* = 8.0 Hz, 1H), 6.95 (d, *J* = 8.4 Hz, 1H), 4.21 (dd, *J* = 9.2, 2.4 Hz, 1H), 3.99 (t, J = 9.2 Hz, 1H), 3.95 (s, 4H), 3.77 (brd, *J* = 9.2 Hz, 1H), 2.36 (s, 3H), 2.32 (s, 3H), 1.05 (s, 9H).

### EXAMPLE 7 (+/-)-3'-((4-(2-Hydroxy-3,3-dimethylbutoxy)-2-methylphenyl)diazenyl)-[1,1'-biphenyl]-4-carboxylic acid ((+/-)-Ic-1)

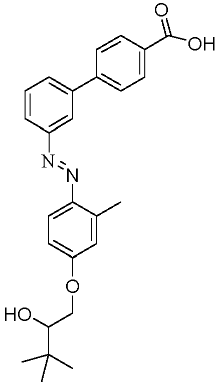

According to method K, from 49 mg of ester ((+/-)-la-1), 36 mg of ((+/-)-Ic-1) (76% yield) were obtained.

¹H NMR (400 MHz, CD₃OD) δ 8.12 (d, *J* = 8.4 Hz, 2H), 8.12-8.10 (m, 1H), 7.84 (ddd, *J* = 8.4, 2.0, 1.2 Hz, 1H), 7.77 (d, *J* = 8.4 Hz, 2H), 7.74 (ddd, *J* = 8.4, 2.0, 1.2 Hz, 1H), 7.72 (d, *J = 8.8* Hz, 2H), 7.58 (t, *J* = 8.4 Hz, 1H), 6.93 (d, *J* = 2.8 Hz, 1H), 6.85 (dd, *J* = 8.8, 2.8 Hz, 1H), 4.21 (dd, *J* = 9.9, 2.6 Hz, 1H), 3.94 (dd, *J* = 9.9, 8.2 Hz, 1H), 3.63 (dd, *J* = 8.2, 2.6 Hz, 1H), 2.72 (s, 3H), 1.02 (s, 9H).

### EXAMPLE 8 (-)-(R) 3'-((4-(2-Hydroxy-3,3-dimethylbutoxy)-2-methylphenyl)diazenyl)-[1,1'-biphenyl]-4-carboxylic acid((-)-Ic-1)

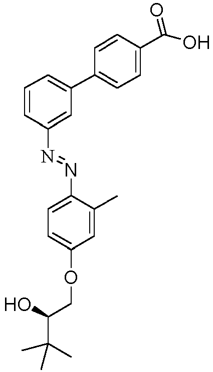

According to method K and J4, from 3.98 mg of ester ((-)-la-1), 3.17 mg of ((-)-lc-1) (82% yield) were obtained 98% ee.
[α] D₂₀ = -15 (c 0.2, MeOH).

### EXAMPLE 9 (+)-(S) 3'-((4-(2-Hydroxy-3,3-dimethylbutoxy)-2-methylphenyl)diazenyl)-[1,1'-biphenyl]-4-carboxylic acid ((+)-lc-1)

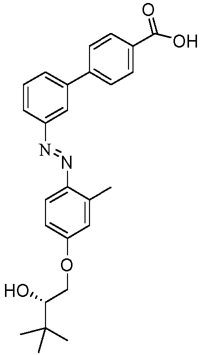

According to method K and J4, from 3.5 mg of ester ((+)-la-1), 2.66 mg of ((+)-lc-1) (78% yield) were obtained. 98% ee.
[α] D₂₀ = +9 (c 0.2, MeOH).

### EXAMPLE 10 (+/-)-3'-((4-(2-Hydroxy-3,3-dimethylbutoxy)-3-methylphenyl)diazenyl)-[1,1'-biphenyl]-4-carboxylic acid ((+/-)-Ic-2)

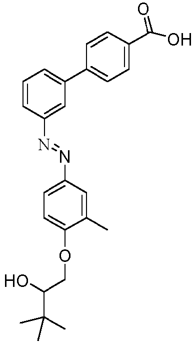

According to method K, from 7.0 mg of ester ((+/-)-la-2), 6.5 mg of ((+/-)-Ic-2) (96% yield) were obtained.

¹H NMR (400 MHz, CD₃OD) δ 8.16-8.14 (m, 1H), 8.14 (d, *J* = 8.4 Hz, 2H), 7.89 (ddd, *J* = 8.0, 1.6, 1.2 Hz, 1H), 7.85-7.80 (m, 2H), 7.82 (d, *J* = 8.4 Hz, 2H), 7.79 (ddd, *J =* 8.0, 1.6, 1.2 Hz, 1H), 7.63 (t, *J* = 8.0 Hz, 1H), 7.08 (d, *J* = 8.6 Hz, 1H), 4.27 (dd, *J =* 10.1, 2.8 Hz, 1H), 4.02 (dd, *J* = 10.1, 7.8 Hz, 1H), 3.69 (dd, *J* = 7.8, 2.8 Hz, 1H), 2.34 (s, 3H), 1.04 (s, 9H).

¹³C NMR (101 MHz, CD₃OD) δ 169.7, 161.7, 154.7, 147.8, 146.1, 142.4, 131.5, 131.3, 130.9, 130.1, 129.0, 128.1, 125.2, 124.9, 123.1, 122.1, 112.0, 78.5, 71.4, 35.2, 26.6, 16.7.

### EXAMPLE 11 (+/-)-3'-((4-(2-Hydroxy-3,3-dimethylbutoxy)-2-methylphenyl)diazenyl)-2-methyl-[1,1'-biphenyl]-4-carboxylic acid ((+/-)-Ic-3)

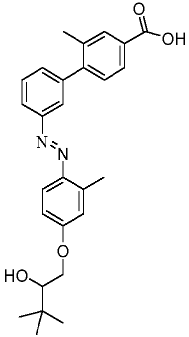

According to method K, from 7.0 mg of ester **((+/-)-la-3),** 6.5 mg of **((+/-)-Ic-3)** (96% yield) were obtained.

¹H NMR (400 MHz, CD₃OD) δ 7.97 (d, *J* = 1.8 Hz, 1H), 7.91 (dd, *J* = 7.8, 1.8 Hz, 1H), 7.87 (ddd, *J* = 7.8, 2.0, 1.2 Hz, 1H), 7.80 (t, *J* = 1.8 Hz, 1H), 7.72 (d, *J* = 9.0 Hz, 1H), 7.60 (t, *J* = 7.8 Hz, 1H), 7.43 (dt, J = 7.8, 1.2 Hz, 1H), 7.36 (d, *J* = 7.8 Hz, 1H), 6.95 (d, *J* = 2.8 Hz, 1H), 6.87 (dd, *J* = 9.0, 2.8 Hz, 1H), 4.22 (dd, *J* = 9.9, 2.6 Hz, 1H), 3.95 (dd, *J* = 9.9, 8.2 Hz, 1H), 3.63 (dd, *J* = 8.2, 2.6 Hz, 1H), 2.71 (s, 3H), 2.35 (s, 3H), 1.02 (s, 9H).

### EXAMPLE 12 (+/-)-3'-((4-(2-Hydroxy-3,3-dimethylbutoxy)-3-methylphenyl)diazenyl)-2-methyl-[1,1'-biphenyl]-4-carboxylic acid. ((+/-)-Ic-4)

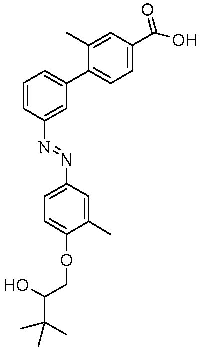

According to method K, from 10.0 mg of ester **((+/-)-la-4),** 1.5 mg of **((+/-)-Ic-4)** (15% yield) were obtained.

¹H NMR (400 MHz, CDCl₃) δ 7.99 (s, 1H), 7.93 (dd, *J* = 8.0, 1.2 Hz, 1H), 7.88 (ddd, *J* = 8.0, 1.8, 1.2 Hz, 1H), 7.83-7.76 (m, 3H), 7.61 (t, *J* = 8.0 Hz, 1H), 7.45 (dt, *J* = 8.0, 1.4 Hz, 1H), 7.38 (d, *J* = 8.0 Hz, 1H), 7.07 (d, *J* = 8.6 Hz, 1H), 4.27 (dd, *J* = 10.1, 2.8 Hz, 1H), 4.01 (dd, *J* = 10.1, 7.8 Hz, 1H), 3.68 (dd, *J* = 7.8, 2.8 Hz, 1H), 2.36 (s, 3H), 2.33 (s, 3H), 1.04 (s, 9H).

### EXAMPLE 13 (+/-)-1-(4-((4'-(Hydroxymethyl)-[1,1'-biphenyl]-3-yl)diazenyl)-3-methylphenoxy)-3,3-dimethylbutan-2-ol ((+/-)-Ib-1)

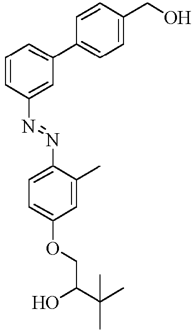

According to method D, from 15.0 mg of ester ((+/-)-la-1), 2.2 mg of ((+/-)-Ib-1) (15% yield) were obtained once it was purified by reverse phase.

¹H NMR (400 MHz, CDCl₃) δ 8.10 (t, *J* = 1.8 Hz, 1H), 7.83 (ddd, *J* = 8.0, 1.8, 1.2 Hz, 1H), 7.76 (d, *J = 9.0* Hz, 1H), 7.72 (ddd, *J* = 8.0, 1.8, 1.2 Hz, 1H), 7.69 (d, *J* = 8.4 Hz, 2H), 7.59 (t, *J =* 8.0 Hz, 1H), 7.48 (d, *J =* 8.4 Hz, 2H), 6.96 (d, *J =* 2.8 Hz, 1H), 6.88 (dd, *J* = 9.0, 2.8 Hz, 1H), 4.68 (s, 2H), 4.58 (brs, 1H), 4.23 (dd, *J* = 9.9, 2.7 Hz, 1H), 3.96 (dd, *J* = 9.9, 8.2 Hz, 1H), 3.64 (dd, *J* = 8.2, 2.7 Hz, 1H), 2.75 (s, 3H), 1.03 (s, 9H).

### Example 14 (-)-1-(4-((4'-(Hydroxymethyl)-[1,1'-biphenyl]-3-yl)diazenyl)-3-methylphenoxy)-3,3-dimethylbutan-2-ol ((-)-Ib-1)

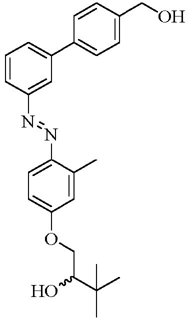

According to method J5, from 13mg of racemic (Ib-1), 6.61mg ((-)-Ib-1) was obtained [α] D₂₀ = -13 (c 0.4, CHCl₃); 100% ee

### Example 15 (+)-1-(4-((4'-(Hydroxymethyl)-[1,1'-biphenyll-3-yl)diazenyl)-3-methylphenoxy)-3,3-dimethylbutan-2-ol ((+)-Ib-1)

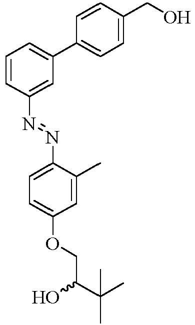

According to method J5, from 13mg of racemic (Ib-1), 5.98mg of ((+)-Ib-1) was obtained.
[α] D₂₀ = +10 (c 0.4, CHCl₃); 100% ee

### EXAMPLE 16 (+/-)-1-(4-((4'-(Hydroxymethyl)-[1,1'-biphenyl]-3-yl)diazenyl)-2-methylphenoxy)-3,3-dimethylbutan-2-ol. ((+/-)-Ib-2)

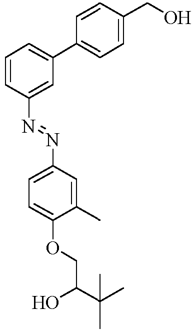

According to method D, from 2.0 mg of ester **((+/-)-la-2),** 1.9 mg of **((+/-)-Ib-2)** (100% yield) were obtained once it was purified by reverse phase.

¹H NMR (400 MHz, CD₃OD) δ 8.10 (t, *J* = 1.8 Hz, 1H), 7.85-7.79 (m, 3H), 7.73 (ddd, *J =* 7.8, 1.8 1.2 Hz, 1H), 7.70 (d, *J = 8.4* Hz, 2H), 7.59 (t, *J = 7.8* Hz, 1H), 7.48 (d, J = 8.4 Hz, 2H), 7.08 (d, *J* = 8.6 Hz, 1H), 4.68 (s, 2H), 4.28 (dd, J = 10.1, 2.8 Hz, 1H), 4.02 (dd, *J* = 10.1, 7.8 Hz, 1H), 3.69 (dd, *J* = 7.8, 2.8 Hz, 1H), 2.03 (s, 3H), 1.04 (s, 9H).

### EXAMPLE 17 (-)-1-(4-((4'-(Hydroxymethyl)-[1,1'-biphenyl]-3-yl)diazenyl)-2-methylphenoxy)-3,3-dimethylbutan-2-ol ((-)-Ib-2)

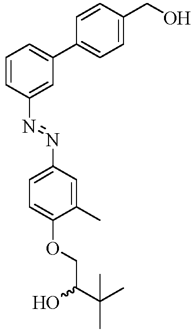

According to method J3, from 4 mg of racemic **(Ib-2),** 0.43mg of **((-)-lb-2)** was obtained.
[α] D₂₀ = -9 (c 0.07, CHCl₃); 100% ee

### EXAMPLE 18 (+)-1-(4-((4'-(Hydroxymethyl)-[1,1'-biphenyl]-3-yl)diazenyl)-2-methylphenoxy)-3,3-dimethylbutan-2-ol ((+)-Ib-2)

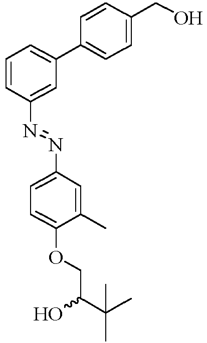

According to method J3, from 4 mg of racemic **(Ib-2),** 0.99 mg of **((+)-Ib-2)** was obtained.
[α] D₂₀ = +3 (c 0.03, CHCl₃); 100%ee.

### EXAMPLE 19 (+/-)-1-(4-((4'-(Hydroxymethyl)-2'-methyl-[1,1'-biphenyl]-3-yl)diazenyl)-3-methylphenoxy)-3,3-dimethylbutan-2-ol ((+/-)-Ib-3)

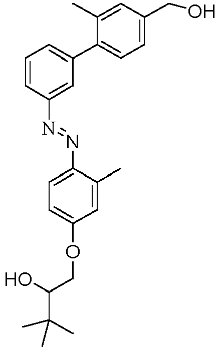

According to method D, from 14.0 mg of ester **((+/-)-la-3),** 5.3 mg of **((+/-)-Ib-3)** (40% yield) were obtained once it was purified by reverse phase.

¹H NMR (400 MHz, CDCl₃) δ 7.75 (ddd, *J* = 8.0, 1.8, 1.2 Hz, 1H), 7.71 (t, *J* = 1.8 Hz, 1H), 7.60 (d, *J* = 8.8 Hz, 1H), 7.42 (t, *J* = 8.0 Hz, 1H), 7.27 (ddd, *J* = 8.0, 1.8, 1.2 Hz, 1H), 7.20-7.15 (m, 3H), 6.74 (d, *J =* 2.8 Hz, 1H), 6.70 (dd, *J* = 8.8, 2.8 Hz, 1H), 4.60 (s, 2H), 4.06 (dd, *J* = 9.2, 2.4 Hz, 1H), 3.82 (t, *J* = 9.2 Hz, 1H), 3.59 (dd, *J* = 9.2, 2.4 Hz, 1H), 2.60 (s, 3H), 2.22 (s, 3H), 0.92 (s, 9H).

### EXAMPLE 20 (+/-)-1-(4-((4'-(Hydroxymethyl)-2'-methyl-[1,1'-biphenyl]-3-yl)diazenyl)-2-methylphenoxy)-3,3-dimethylbutan-2-ol ((+/-)-Ib-4)

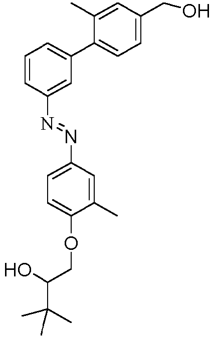

According to method D, from 10.0 mg of ester **((+/-)-la-4),** 2.4 mg of **((+/-)-Ib-4)** (25% yield) were obtained once it was purified by reverse phase.

¹H NMR (400 MHz, CD₃OD) δ 7.84 (ddd, *J* = 7.8, 1.8, 1.0 Hz, 1H), 7.80 (dd, *J* = 8.6, 2.3 Hz, 1H), 7.77-7.75 (m, 2H), 7.57 (t, *J* = 7.8 Hz, 1H), 7.41 (dt, J = 7.6, 1.2 Hz, 1H), 7.31 (s, 1H), 7.27-7.23 (m, 2H), 7.07 (d, *J* = 8.6 Hz, 1H), 4.64 (s, 2H), 4.26 (dd, J *=* 10.0, 2.9 Hz, 1H), 4.01 (dd, *J* = 10.0, 7.8 Hz, 1H), 3.68 (dd, *J* = 7.8, 2.8 Hz, 1H), 2.33 (s, 3H), 2.31 (s, 3H), 1.04 (s, 9H).

### EXAMPLE 21 Methyl-(3'-(4-(2-hydroxy-3,3-dimethylbutoxy)-3-methylphenyl)diazenyl)-[1,1'-biphenyl]-4-carbonyl)-L-serinate (diastereomeric mixture) (Id-1)

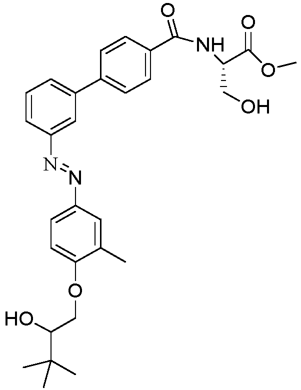

According to method M, from 3.0 mg of acid **((+/-)-Ic-2),** and 1.19 mg of (S)-methyl 2-amino-3-hydroxypropanoate.HCl, 3.48 mg of diastereomeric mixture **(Id-1)** (94% yield) were obtained once it was purified by reverse phase.

¹H NMR (400 MHz, CD₃OD) δ 8.16 (t, *J* = 1.9 Hz, 1H), 8.02 (d, *J* = 8.4 Hz, 2H), 7.89 (ddd, *J* = 7.8, 1.8, 1.0 Hz, 1H), 7.85 (d, *J* = 8.4 Hz, 2H), 7.84-7.79 (m, 3H), 7.64 (t, *J* = 7.8 Hz, 1H), 7.09 (d, *J* = 8.6 Hz, 1H), 4.77 (dd, *J* = 5.2, 4.2 Hz, 1H), 4.58 (s, 1H), 4.28 (dd, *J* = 10.0, 2.8 Hz, 1H), 4.04 (dd, *J* = 11.4, 5.2 Hz, 1H), 4.02 (dd, *J* = 10.0, 7.9 Hz, 1H), 3.97 (dd, J = 11.4, 4.2 Hz, 1H), 3.80 (s, 3H), 3.69 (dd, *J* = 7.9, 2.8 Hz, 1H), 2.35 (s, 3H), 1.04 (s, 9H).

### EXAMPLE 22 (3'-(4-(2-Hydroxy-3,3-dimethylbutoxy)-3-methylphenyl)diazenyl)-[1,1'-biphenyl]-4-carbonyl)-L-serine) (diastereomeric mixture) (Id-2)

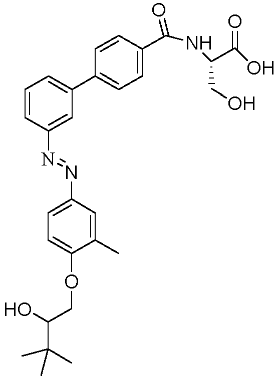

According to method K, from 3.1 mg of ester **(Id-1),** 2.5 mg of diastereomeric mixture **(Id-2)** (83% yield) were obtained.

¹H NMR (400 MHz, CD₃OD) δ 8.16 (t, *J* = 1.6 Hz, 1H), 8.03 (d, *J* = 8.5 Hz, 2H), 7.89 (ddd, *J* = 8.0, 1.6, 1.1 Hz, 1H), 7.90-7.79 (m, 5H), 7.64 (t, *J =* 8.0 Hz, 1H), 7.09 (d, *J* = 8.8 Hz, 1H), 4.73-4.69 (m, 1H), 4.28 (dd, *J* = 10.0, 2.8 Hz, 1H), 4.06-3.96 (m, 3H), 3.69 (dd, *J* = 7.8, 2.8 Hz, 1H), 2.35 (s, 3H), 1.04 (s, 9H).

### EXAMPLE 23 1-(4-((4'-(((2,2-Dimethyl-1,3-dioxolan-4-yl)methoxy)methyl)-[1,1'-biphenyl]-3-yl)diazenyl)-3-methylphenoxy)-3,3-dimethylbutan-2-ol (diastereomeric mixture) (le-1)

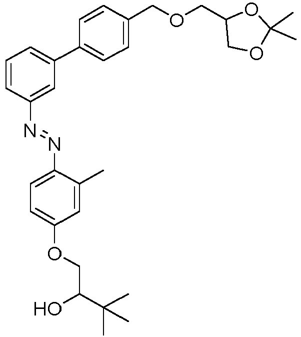

According to method G, from 4.0 mg of nitroso **((+/-)-6-6)** and 2.5 mg of amine **((+/-)-10-1)** in AcOH:DCM (0.04 M), 2.9 mg of diastereomeric mixture **(le-1)** (33% yield) were obtained.

¹H NMR (400 MHz, CD₃OD) δ 8.10 (t, *J =* 1.6 Hz, 1H), 7.83 (ddd, J= 7.6, 1.6, 1.2 Hz, 1H), 7.74 (d, *J* = 9.0 Hz, 1H), 7.73 (ddd, *J* = 7.6, 1.6, 1.2 Hz, 1H), 7.70 (d, *J* = 8.4 Hz, 2H), 7.59 (t, *J =* 7.6 Hz, 1H), 7.48 (d, *J =* 8.4 Hz, 2H), 6.96 (d, *J =* 2.8 Hz, 1H), 6.88 (dd, *J* = 9.0, 2.8 Hz, 1H), 4.64 (s, 2H), 4.32 (tt, *J* = 6.4, 5.2 Hz, 1H), 4.24 (dd, J = 9.9, 2.7 Hz, 1H), 4.08 (dd, *J* = 8.3, 6.4 Hz, 1H), 3.96 (dd, *J* = 9.9, 8.2 Hz, 1H), 3.77 (dd, *J* = 8.3, 6.4 Hz, 1H), 3.64 (dd, *J* = 8.2, 2.7 Hz, 1H), 3.59 (dd, *J* = 9.9, 5.2 Hz, 1H), 3.56 (dd, *J* = 9.9, 5.2 Hz, 1H), 1.40 (s, 3H), 1.35 (s, 3H), 1.29 (s, 3H), 1.03 (s, 9H).

### EXAMPLE 24 1-(4-((4'-(((2,2-Dimethyl-1,3-dioxolan-4-yl)methoxy)methyl)-[1,1'-biphenyl]-3-yl)diazenyl)-2-methylphenoxy)-3,3-dimethylbutan-2-ol (diastereomeric mixture) (le-2)

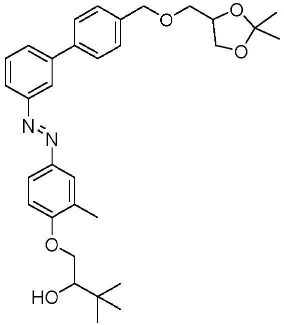

According to method G, from 4.0 mg of nitroso **((+/-)-6-6)** and 2.5 mg of amine **((+/-)-10-2)** in AcOH:DCM (0.05 M), 3.2 mg of diastereomeric mixture **(le-2)** (54% yield) were obtained.

¹H NMR (400 MHz, CD₃OD) δ 8.12 (t, *J* = 2.0 Hz, 1H), 7.86 (ddd, *J* = 8.0, 2.0, 1.4 Hz, 1H), 7.85 (dd, *J* = 8.4, 2.4 Hz, 1H), 7.82 (d, *J* = 2.4 Hz, 1H), 7.76 (ddd, *J* = 8.0, 2.0, 1.4 Hz, 1H), 7.73 (d, *J* = 8.2 Hz, 2H), 7.62 (t, *J* = 8.0 Hz, 1H), 7.50 (d, *J* = 8.2 Hz, 2H), 7.11 (d, *J = 8.4* Hz, 1H), 4.66 (s, 2H), 4.35 (tt, *J* = 6.4, 5.2 Hz, 2H), 4.30 (dd, *J =* 10.0, 2.8 Hz, 1H), 4.10 (dd, *J* = 8.2, 6.5 Hz, 1H), 4.04 (dd, *J* = 10.0, 7.8 Hz, 1H), 3.79 (dd, *J = 8.3,* 6.4 Hz, 1H), 3.71 (dd, *J* = 7.8, 2.8 Hz, 1H), 3.61 (dd, *J* = 10.0, 5.2 Hz, 1H), 3.58 (dd, J = 10.0, 5.2 Hz, 1H), 2.37 (s, 3H), 1.42 (s, 3H), 1.37 (s, 3H), 1.06 (s, 9H).

### EXAMPLE 25 3-((3'-((4-(2-Hydroxy-3,3-dimethylbutoxy)-2-methylphenyl)diazenyl)-[1,1'-biphenyl]-4-yl)methoxy)propane-1,2-diol (diastereomeric mixture) (le-3)

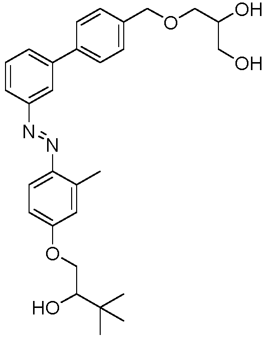

According to method N, from 1.4 mg of acetal **(le-1),** 1.2 mg of diastereomeric mixture **(Ie-3)** (96% yield) were obtained.

¹H NMR (400 MHz, CD₃OD) δ 8.12 (t, *J* = 1.8 Hz, 1H), 7.86 (ddd, *J* = 8.0, 1.8, 1.2 Hz, 1H), 7.78 (d, *J* = 9.0 Hz, 1H), 7.75 (ddd, *J* = 8.0, 1.8, 1.2 Hz, 1H), 7.72 (d, *J* = 8.1 Hz, 2H), 7.61 (t, *J =* 8.0 Hz, 1H), 7.52 (d, *J* = 8.1 Hz, 2H), 6.99 (d, *J* = 2.8 Hz, 1H), 6.91 (dd, *J* = 9.0, 2.8 Hz, 1H), 4.64 (s, 2H), 4.26 (dd, *J* = 10.0, 2.6 Hz, 1H), 3.99 (dd, J *=* 10.0, 8.3 Hz, 1H), 3.84 (tt, *J = 6.0,* 4.8 Hz, 1H), 3.68-3.55 (m, 5H), 2.77 (s, 3H), 1.05 (s, 9H).

### EXAMPLE 26 3-((3'-((4-(2-Hydroxy-3,3-dimethylbutoxy)-3-methylphenyl)diazenyl)-[1,1'-biphenyl]-4-yl)methoxy)propane-1,2-diol (diastereomeric mixture) (le-4)

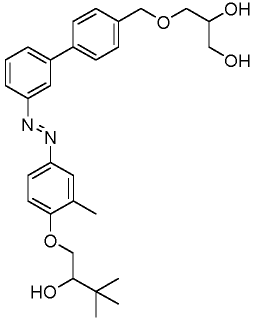

According to method N, from 2.7 mg of acetal **(le-2),** 1.9 mg of diastereomeric mixture **(Ie-4)** (83% yield) were obtained.

¹H NMR (400 MHz, CD₃OD) δ 8.10 (t, *J* = 1.8 Hz, 1H), 7.86-7.79 (m, 3H), 7.74 (ddd, *J* = 8.0, 2.0, 1.2 Hz, 1H), 7.70 (d, *J = 8.2* Hz, 2H), 7.60 (t, *J* = 8.0 Hz, 1H), 7.49 (d, *J* = 8.2 Hz, 2H), 7.09 (d, *J* = 8.7 Hz, 1H), 4.63 (s, 2H), 4.28 (dd, J = 10.0, 2.9 Hz, 1H), 4.02 (dd, *J* = 10.0, 7.8 Hz, 1H), 3.83 (tt, *J = 6.0,* 52 Hz, 1H), 3.76-3.51 (m, 5H), 2.34 (s, 3H), 1.04 (s, 9H).

### EXAMPLE 27 (+/-)-Ethyl 4'-((4-(2-hydroxy-3,3-dimethylbutoxy)-2-methylphenyl)diazenyl)-[1,1'-biphenyl]-4-carboxylate ((+/-)-Ia-5)

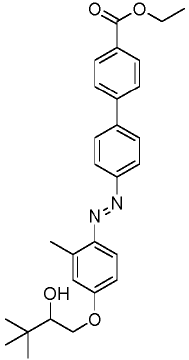

According to method G, from 26 mg of nitroso (6-3) and 21 mg of amine **((+/-)-10-1)** in AcOH (0.1 M), 6.6 mg of **((+/-)-Ia-5)** (15% yield) were obtained.

¹H NMR (400 MHz, CD₃OD) δ 8.02 (d, *J* = 8.4 Hz, 2H), 7.86 (d, *J* = 8.4 Hz, 2H), 7.75 (d, *J* = 8.4 Hz, 2H), 7.73 (d, *J* = 8.4 Hz, 2H), 7.63 (d, *J* = 9.0 Hz, 1H), 6.85 (d, *J* = 2.8 Hz, 1H), 6.77 (dd, *J* = 9.0, 2.8 Hz, 1H), 4.29 (q, *J* = 7.1 Hz, 2H), 4.13 (dd, *J* = 9.9, 2.6 Hz, 1H), 3.85 (dd, *J* = 9.9, 8.2 Hz, 1H), 3.53 (dd, *J* = 8.2, 2.6 Hz, 1H), 2.64 (s, 3H), 1.31 (t, *J* = 7.1 Hz, 3H), 0.92 (s, 9H).

### EXAMPLE 28 (+/-)-Ethyl 4'-((4-(2-hydroxy-3,3-dimethylbutoxy)-3-methylphenyl)diazenyl)-[1,1'-biphenyl]-4-carboxylate ((+/-)-la-6)

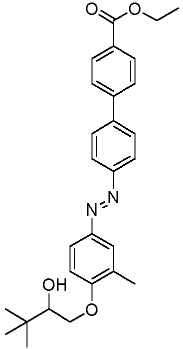

According to method G, from 38 mg of nitroso (6-3) and 30 mg of amine **((+/-)-10-2)** in AcOH (0.1 M), 12.2 mg of **(la-6)** (20% yield) were obtained.

¹H NMR (400 MHz, CD₃OD) δ 8.13 (d, *J* = 8.4 Hz, 2H), 7.97 (d, *J* = 8.4 Hz, 2H), 7.87 (d, J = 8. Hz, 2H), 7.84 (d, *J* = 8.4 Hz, 2H), 7.82 (dd, *J* = 8.6, 2.4 Hz, 1H), 7.79 (d, *J =* 2.4 Hz, 1H), 7.09 (d, *J* = 8.6 Hz, 1H), 4.40 (q, *J* = 7.1 Hz, 2H), 4.28 (dd, *J* = 10.1, 2.8 Hz, 1H), 4.02 (dd, *J =* 10.1, 7.8 Hz, 1H), 3.69 (dd, *J* = 7.8, 2.8 Hz, 1H), 2.35 (s, 3H), 1.42 (t, *J* = 7.1 Hz, 3H), 1.04 (s, 9H).

### EXAMPLE 29 (+/-)-Methyl 4'-((4-(2-hydroxy-3,3-dimethylbutoxy)-2-methylphenyl)diazenyl)-2-methyl-[1,1'-biphenyl]-4-carboxylate ((+/-)-Ia-7)

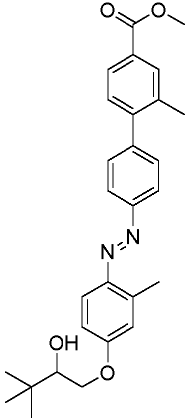

According to method G, from 12.6 mg of nitroso **(6-4)** and 10.0 mg of amine **((+/-)-10-1)** in AcOH (0.05 M), 12.2 mg of **((+/-)-la-7)** (27% yield) were obtained.

¹H NMR (400 MHz, CD₃OD) δ 7.98 (d, *J =* 0.8 Hz, 1H), 7.95 (d, *J =* 8.4 Hz, 2H), 7.93 (dd, *J* = 8.0, 0.8 Hz, 1H), 7.75 (d, *J =* 8.9 Hz, 1H), 7.45 (d, *J = 8.4* Hz, 2H), 7.35 (d, *J* = 8.0 Hz, 1H), 6.87 (d, *J* = 2.8 Hz, 1H), 6.83 (dd, *J* = 8.9, 2.8 Hz, 1H), 4.18 (dd, *J =* 9.2, 2.4 Hz, 1H), 3.96 (dd, *J* = 9.2, 8.8 Hz, 1H), 3.95 (s, 3H), 3.72 (dd, *J =* 8.8, 2.4 Hz, 1H), 2.75 (s, 3H), 2.37 (s, 3H), 1.04 (s, 9H).

### EXAMPLE 30 (+/-)-Ethyl 4'-((4-(2-Hydroxy-3,3-dimethylbutoxy)-2-methylphenyl)diazenyl)-3'-methyl-[1,1'-biphenyl]-4-carboxylate ((+/-)-la-8)

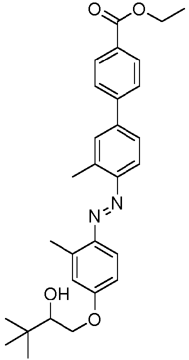

According to method G, from 23.5 mg of nitroso **(6-5)** and 27.7 mg of amine **((+/-)-10-1)** in AcOH (0.05 M), 25.8 mg of **((+/-)-la-8)** (68% yield) were obtained.

¹H NMR (400 MHz, CD₃OD) δ 8.10 (d, *J* = 8.5 Hz, 2H), 7.80 (d, *J* = 8.5 Hz, 2H), 7.71 (d, *J =* 8.8 Hz, 1H), 7.69 (d, *J = 9.2* Hz, 1H), 7.68 (d, *J =* 2.0 Hz, 1H), 7.58 (dd, *J =* 9.2, 2.0 Hz, 1H), 6.95 (d, *J = 2.8* Hz, 1H), 6.87 (dd, *J* = 8.8, 2.8 Hz, 1H), 4.39 (q, *J =* 7.1 Hz, 2H), 4.23 (dd, *J =* 9.9, 2.6 Hz, 1H), 3.96 (dd, *J =* 9.9, 8.2 Hz, 1H), 3.64 (dd, *J* = 8.2, 2.6 Hz, 1H), 2.77 (s, 3H), 2.74 (s, 3H), 1.41 (t, *J* = 7.1 Hz, 3H), 1.03 (s, 9H).

### EXAMPLE 31 (+/-)-Ethyl 4'-((4-(2-hydroxy-3,3-dimethylbutoxy)-3-methylphenyl)diazenyl)-3'-methyl-[1,1'-biphenyl]-4-carboxylate ((+/-)-la-9)

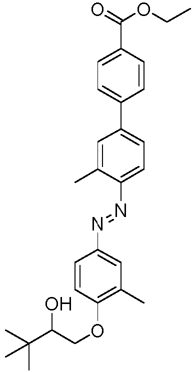

According to method G, from 26.2 mg of nitroso **(6-5)** and 21.7 mg of amine **((+/-)-10-**2) in AcOH (0.05 M), 32.9 mg of **((+/-)-la-9)** (71% yield) were obtained.

¹H NMR (400 MHz, CD₃OD) δ 8.10 (d, *J* = 8.5 Hz, 2H), 7.81 (d, *J* = 8.8 Hz, 1H), 7.80 (d, *J =* 8.5 Hz, 2H), 7.76 (d, *J* = 1.8 Hz, 1H), 7.69 (d, *J* = 2.0 Hz, 1H), 7.68 (d, *J* = 8.4 Hz, 1H), 7.59 (dd, *J =* 8.4, 2.0 Hz, 1H), 7.07 (d, *J =* 8.8 Hz, 1H), 4.39 (q, *J =* 7.1 Hz, 2H), 4.27 (dd, *J =* 10.1, 2.8 Hz, 1H), 4.01 (dd, *J =* 10.1, 7.8 Hz, 1H), 3.69 (dd, *J* = 7.8, 2.8 Hz, 1H), 2.77 (s, 3H), 2.34 (s, 3H), 1.41 (t, *J* = 7.1 Hz, 3H), 1.04 (s, 9H).

### EXAMPLE 32 (+/-)-4'-((4-(2-Hydroxy-3,3-dimethylbutoxy)-2-methylphenyl)diazenyl)-[1,1'-biphenyl]-4-carboxylic acid ((+/-)-lc-5)

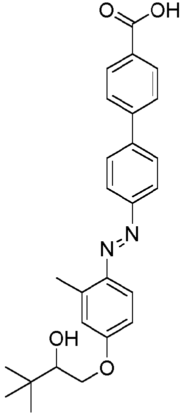

According to method K, from 7.0 mg of ester **((+/-)-la-5),** 6.5 mg of **((+/-)-Ic-5)** (96% yield) were obtained.

¹H NMR (400 MHz, CD₃OD) δ 8.03 (d, *J =* 8.4 Hz, 2H), 7.88 (d, *J =* 8.6 Hz, 2H), 7.77 (d, *J* = 8.6 Hz, 2H), 7.73 (d, *J =* 8.4 Hz, 2H), 7.64 (d, *J =* 9.0 Hz, 1H), 6.87 (d, *J* = 2.8 Hz, 1H), 6.79 (dd, *J =* 9.0, 2.8 Hz, 1H), 4.14 (dd, *J =* 10.0, 2.5 Hz, 1H), 3.86 (dd, *J =* 10.0, 8.2 Hz, 1H), 3.57 (dd, *J =* 8.2, 2.5 Hz, 1H), 2.65 (s, 3H), 0.93 (s, 9H).

### EXAMPLE 33 (+/-)-4'-((4-(2-Hydroxy-3,3-dimethylbutoxy)-3-methylphenyl)diazenyl)-[1,1'-biphenyl]-4-carboxylic acid ((+/-)-Ic-6)

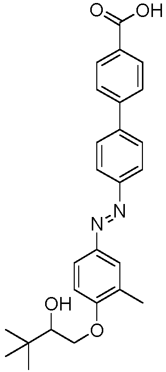

According to method K, from 4.3 mg of ester **((+/-)-la-6),** 4.0 mg of **((+/-)-Ic-6)** (99% yield) were obtained.

¹H NMR (400 MHz, CD₃OD) δ 8.03 (d, *J =* 8.5 Hz, 2H), 7.87 (d, *J* = 8.6 Hz, 2H), 7.77 (d, *J =* 8.6 Hz, 2H), 7.73 (d, *J =* 8.5 Hz, 2H), 7.72 (dd, *J =* 8.8, 2.8 Hz, 1H), 7.69 (d, *J* = 2.8 Hz, 1H), 6.99 (d, *J* = 8.8 Hz, 1H), 4.18 (dd, *J* = 10.2, 2.4 Hz, 1H), 4.12 (dd, *J* = 6.2, 2.4 Hz, 1H), 3.92 (dd, *J =* 10.2, 6.2 Hz, 1H), 2.25 (s, 3H), 0.95 (s, 9H).

### EXAMPLE 34 (+/-)-4'-((4-(2-Hydroxy-3,3-dimethylbutoxy)-2-methylphenyl)diazenyl)-3'-methyl-[1,1'-biphenyl]-4-carboxylic acid ((+/-)-Ic-7)

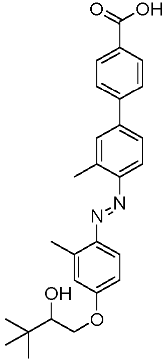

According to method K, from 9.0 mg of ester **((+/-)-Ia-8),** 6.7 mg of **((+/-)-Ic-7)** (79% yield) were obtained.

¹H NMR (400 MHz, CD₃OD) δ 8.03 (d, *J =* 8.4 Hz, 2H), 7.71 (d, *J =* 8.4 Hz, 2H), 7.63 (d, *J =* 7.6 Hz, 1H), 7.62-7.60 (m, 2H), 7.50 (dd, *J =* 7.6, 2.0 Hz, 1H), 6.86 (d, *J =* 2.8 Hz, 1H), 6.79 (dd, *J =* 9.0, 2.8 Hz, 1H), 4.14 (dd, *J* = 9.9, 2.6 Hz, 1H), 3.87 (dd, *J =* 9.9, 8.2 Hz, 1H), 3.55 (dd, *J =* 8.2, 2.6 Hz, 1H), 2.69 (s, 3H), 2.66 (s, 3H), 0.94 (s, 9H).

### EXAMPLE 35 (+/-)-4'-((4-(2-Hydroxy-3,3-dimethylbutoxy)-3-methylphenyl)diazenyl)-3'-methyl-[1,1'-biphenyl]-4-carboxylic acid ((+/-)-Ic-8)

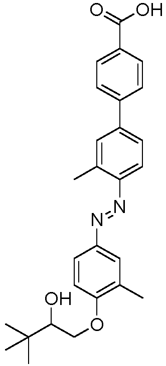

According to method K, from 13.0 mg of ester **((+/-)-Ia-9),** 12.2 mg of **((+/-)-Ic-8)** (99% yield) were obtained.

¹H NMR (400 MHz, CD₃OD) δ 8.14 (d, *J* = 8.5 Hz, 2H), 7.83 (dd, *J =* 8.4, 2.4 Hz, 2H), 7.82 (d, *J* = 8.5 Hz, 2H), 7.79 (d, *J* = 2.0 Hz, 1H), 7.73 (d, *J* = 2.4 Hz, 1H), 7.71 (d, *J* = 8.4 Hz, 1H), 7.62 (dd, *J* = 8.4, 2.0 Hz, 1H), 7.10 (d, *J =* 8.4 Hz, 1H), 4.29 (dd, *J =* 10.0, 2.8 Hz, 1H), 4.04 (dd, *J =* 10.0, 7.8 Hz, 1H), 3.71 (dd, *J =* 7.8, 2.8 Hz, 1H), 2.80 (s, 3H), 2.37 (s, 3H), 1.06 (s, 9H).

### EXAMPLE 36 (+/-)-1-(4-((4'-(Hydroxymethyl)-[1,1'-biphenyl]-4-yl)diazenyl)-3-methylphenoxy)-3,3-dimethylbutan-2-ol ((+/-)-Ib-5)

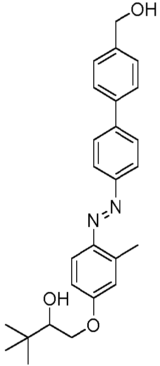

According to method D, from 2.0 mg of ester ((+/-)-la-5), 1.8 mg of ((+/-)-Ib-5) (99% yield) were obtained.

HPLC-MS: retention time= 4.61 min; m/z: 419 [M+1]⁺.

### EXAMPLE 37 (+/-)-1-(4-((4'-(Hydroxymethyl)-[1,1'-biphenyl]-4-yl)diazenyl)-2-methylphenoxy)-3,3-dimethylbutan-2-ol ((+/-)-Ib-6)

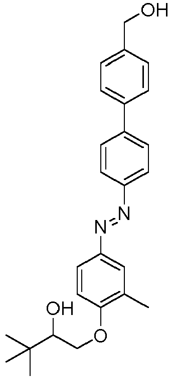

According to method D, from 6.0 mg of ester ((+/-)-la-6), 5.4 mg of ((+/-)-Ib-6) (99% yield) were obtained.

¹H NMR (400 MHz, CD₃OD) δ 7.84 (d, *J =* 8.6 Hz, 2H), 7.74-7.66 (m, 2H), 7.69 (d, *J* = 8.6 Hz, 2H), 7.60 (d, *J =* 8.2 Hz, 2H), 7.37 (d, *J* = 8.2 Hz, 2H), 6.98 (d, *J =* 8.6 Hz, 1H), 4.57 (s, 2H), 4.17 (dd, *J =* 10.0, 2.9 Hz, 1H), 3.92 (dd, *J =* 10.0, 7.8 Hz, 1H), 3.59 (dd, *J* = 7.8*,* 2.8 Hz, 1H), 2.24 (s, 3H), 0.94 (s, 9H).

### EXAMPLE 38 (+/-)-1-(4-((4'-(Hydroxymethyl)-2'-methyl-[1,1'-biphenyl]-4-yl)diazenyl)-3-methylphenoxy)-3,3-dimethylbutan-2-ol ((+/-)-Ib-7)

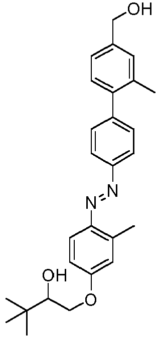

According to method D, from 2.3 mg of ester **((+/-)-Ia-7),** 2.1 mg of **((+/-)-Ib-7)** (99% yield) were obtained.

¹H NMR (400 MHz, CD₃OD) δ 7.82 (d, *J =* 8.5 Hz, 2H), 7.63 (d, *J =* 9.0 Hz, 1H), 7.36 (d, *J =* 8.5 Hz, 2H), 7.20 (s, 1H), 7.15 (s, 2H), 6.86 (d, *J =* 2.8 Hz, 1H), 6.78 (dd, *J =* 9.0, 2.8 Hz, 1H), 4.53 (s, 2H), 4.13 (dd, *J* = 9.9, 2.5 Hz, 1H), 3.86 (dd, *J* = 9.9, 8.2 Hz, 1H), 3.54 (dd, *J* = 8.2, 2.5 Hz, 1H), 2.64 (s, 3H), 2.22 (s, 3H), 0.93 (s, 9H).

### EXAMPLE 39 (+/-)-1-(4-((4'-(Hydroxymethyl)-3-methyl-[1,1'-biphenyl]-4-yl)diazenyl)-2-methylphenoxy)-3,3-dimethylbutan-2-ol ((+/-)-Ib-8)

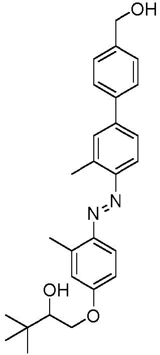

According to method D, from 15.0 mg of ester **((+/-)-Ia-8),** 13.6 mg of **((+/-)-Ib-8)** (99% yield) were obtained.

¹H NMR (400 MHz, CD₃OD) δ 7.80 (dd, *J =* 8.8, 2.4 Hz, 1H), 7.76 (d, *J =* 2.4 Hz, 1H), 7.67 (d, *J* = 8.4 Hz, 1H), 7.67 (dd, *J* = 8.0 Hz, 2H), 7.62 (d, *J =* 2.0 Hz, 1H), 7.52 (dd, *J* = 8.5, 2.0 Hz, 1H), 7.47 (d, *J* = 8.0 Hz, 2H), 7.07 (d, *J* = 8.5 Hz, 1H), 4.27 (dd, *J =* 10.1, 2.9 Hz, 1H), 4.03 (dd, *J =* 10.1, 7.8 Hz, 1H), 3.71 (dd, *J* = 7.8, 2.9 Hz, 1H), 2.77 (s, 3H), 2.36 (s, 3H), 1.06 (s, 9H).

### EXAMPLE 40 (+/-)-1-(4-((4'-(Hydroxymethyl)-3-methyl-[1,1'-biphenyl]-4-yl)diazenyl)-3-methylphenoxy)-3,3-dimethylbutan-2-ol ((+/-)-Ib-9)

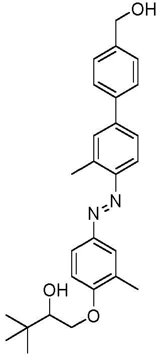

According to method D, from 13.0 mg of ester **((+/-)-Ia-9),** 5.4 mg of **((+/-)-Ib-9)** (46% yield) were obtained.

¹H NMR (400 MHz, CD₃OD) δ 7.72 (d, *J =* 9.0 Hz, 1H), 7.69 (d, *J =* 8.4 Hz, 1H), 7.68 (d, *J =* 8.3 Hz, 2H), 7.62 (d, *J = 2.0* Hz, 1H), 7.53 (dd, *J =* 8.4, 2.0 Hz, 1H), 7.48 (d, *J* = 8.3 Hz, 2H), 6.96 (d, *J* = 2.8 Hz, 1H), 6.88 (dd, *J* = 9.0, 2.8 Hz, 1H), 4.69 (s, 2H), 4.24 (dd, *J =* 9.9, 2.6 Hz, 1H), 3.98 (dd, *J =* 9.9, 8.1 Hz, 1H), 3.66 (dd, *J =* 8.1, 2.6 Hz, 1H), 2.78 (s, 3H), 2.76 (s, 3H), 1.05 (s, 9H).

### EXAMPLE 41 (+/-)-2-(4'-((4-(2-Hydroxy-3,3-dimethylbutoxy)-2-methylphenyl)diazenyl)-3'-methyl-[1,1'-biphenyl]-4-yl)acetic acid ((+/-)-Ic-9)

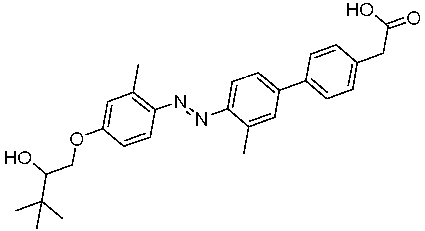

According to method E, from 62 mg of phenol (7-1), 63 µL of 2-(tert-butyl)oxirane and 95 mg of K₂CO₃, 0.6 mL of DMF, 28 mg of ((+/-)-lc-9) were obtained (35% yield) once it was purified by reverse phase.

¹H NMR (400 MHz, CDCl₃) δ 7.72 (d, *J =* 8.9 Hz, 1H), 7.69 (d, *J =* 8.4 Hz, 1H), 7.62 (d, *J* = 8.0 Hz, 2H), 7.54 (d, *J* = 2.1 Hz, 1H), 7.47 (dd, *J* = 8.4, 2.1 Hz, 1H), 7.39 (d, *J* = 8.0 Hz, 2H), 6.86 (d, *J* = 2.8 Hz, 1H), 6.82 (dd, *J =* 8.9, 2.8 Hz, 1H), 4.18 (dd, *J =* 9.2, 2.4 Hz, 1H), 3.94 (t, *J =* 9.2 Hz, 1H), 3.72 (s, 2H) 3.72 (dd, *J* = 9.2, 2.4 Hz, 1H), 2.77 (s, 3H), 2.76 (s, 3H), 1.04 (s, 9H).

### EXAMPLE 42 (+/-)-2-(4'-((4-(2-Hydroxy-3,3-dimethylbutoxy)-3-methylphenyl)diazenyl)-3'-methyl-[1,1'-biphenyl]-4-yl)acetic acid ((+/-)-lc-10)

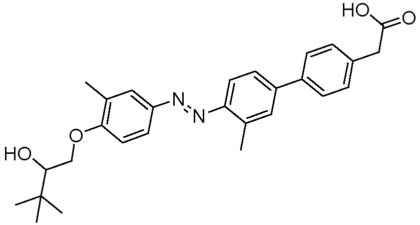

According to method E, from 66 mg of phenol **(7-2),** 67 µL of 2-(tert-butyl)oxirane and 95 mg of K₂CO₃, 0.6 mL of DMF, 3.2 mg of **((+/-)-Ic-10)** were obtained (15% yield) once it was purified by reverse phase.

¹H NMR (400 MHz, CDCl₃) δ 7.81 (dd, *J* = 8.4, 2.5 Hz, 1H), 7.78 (d, *J =* 2.5 Hz, 1H), 7.68 (d, *J* = 8.4 Hz, 1H), 7.62 (d, *J* = 8.2 Hz, 2H), 7.54 (d, *J* = 8.4, 2.0 Hz, 1H), 7.48 (dd, *J* = 8.4, 2.0 Hz, 1H), 7.39 (d, *J =* 8.2 Hz, 2H), 6.95 (d, *J* = 8.4 Hz, 1H), 4.22 (dd, *J* = 9.0*,* 2.6 Hz, 1H), 3.99 (t, *J* = 9.0 Hz, 1H), 3.78 (dd, *J* = 9.0, 2.6 Hz, 1H), 3.73 (s, 2H), 2.77 (s, 3H), 2.33 (s, 3H), 1.05 (s, 9H).

### EXAMPLE 43 (+/-)-Methyl 2-(4'-((4-(2-hydroxy-3,3-dimethylbutoxy)-2-methylphenyl)diazenyl)-3'-methyl-[1,1'-biphenyl]-4-yl)acetate ((+/-)-la-10)

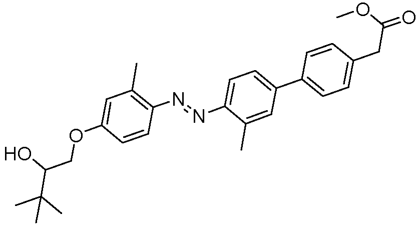

According to method R, from 12 mg of acid **((+/-)-Ic-9),** 12 mg of **((+/-)-Ia-10)** were obtained (100% yield).

¹H NMR (400 MHz, CDCl₃) δ 7.75-7.69 (m, 2H), 7.61 (d, *J =* 8.0 Hz, 2H), 7.54 (d, *J =* 2.0 Hz, 1H), 7.48 (d, *J* = 7.1 Hz, 1H), 7.37 (d, *J =* 8.0 Hz, 2H), 6.86 (s, 1H), 6.82 (brs, 1H), 4.18 (dd, *J =* 9.2, 2.3 Hz, 1H), 3.95 (dd, *J* = 9.2, 8.8 Hz, 1H), 4.18 (dd, *J* = 8.8, 2.3 Hz, 1H), 3.73 (s, 3H), 3.69 (s, 2H), 2.78 (s, 3H), 2.76 (s, 3H), 1.04 (s, 9H).

### EXAMPLE 44 (+/-)-Methyl 2-(4'-((4-(2-hydroxy-3,3-dimethylbutoxy)-3-methylphenyl)diazenyl)-3'-methyl-[1,1'-biphenyl]-4-yl)acetate ((+/-)-la-11)

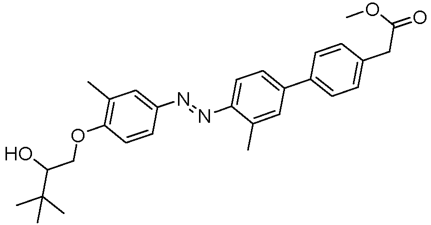

According to method R, from 5.0 mg of acid **((+/-)-Ic-10),** 5.1 mg of **((+/-)-Ia-11)** were obtained (100% yield).

¹H NMR (400 MHz, CDCl₃) δ 7.82 (d, *J* = 7.8 Hz, 1H), 7.79 (s, 1H), 7.70 (d, *J* = 8.0 Hz, 1H), 7.61 (d, *J* = 8.0 Hz, 2H), 7.55 (s, 1H), 7.48 (d, *J =* 7.8 Hz, 1H), 7.37 (d, *J =* 8.0 Hz, 2H), 6.95 (d, *J* = 8.0 Hz, 1H), 4.22 (dd, *J =* 9.0, 2.4 Hz, 1H), 3.99 (t, *J* = 9.0 Hz, 1H), 3.78 (dd, *J* = 9.0, 2.4 Hz, 1H), 3.72 (s, 3H), 3.69 (s, 2H), 2.77 (s, 3H), 2.34 (s, 3H), 1.05 (s, 9H).

### EXAMPLE 45 (+/-)-1-(4-((4'-(2-Hydroxyethyl)-3-methyl-[1,1'-biphenyl]-4-yl)diazenyl)-3-methylphenoxy)-3,3-dimethylbutan-2-ol ((+/-)-Ib-10)

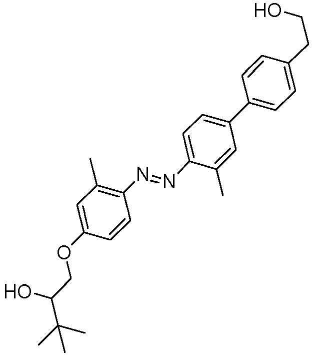

According to method D, from 16.0 mg of **((+/-)-la-10)** in THF (0.01 M), 4.4 mg of **((+/- )-Ib-10)** (35% yield) were obtained, once it was purified by reverse phase.

¹H NMR (400 MHz, CDCl₃) δ 7.75 (d, *J* = 8.8 Hz, 1H), 7.72 (d, *J* = 8.4 Hz, 1H), 7.63 (d, *J* = 8.2 Hz, 2H), 7.57 (d, *J* = 2.4 Hz, 1H), 7.50 (dt, *J* =8.4, 2.4 Hz, 1H), 7.35 (d, *J =* 8.2 Hz, 1H), 6.88 (d, *J* = 2.4 Hz, 1H), 6.84 (dd, *J =* 8.8, 2.4 Hz, 1H), 4.21 (dd, *J* = 8.8, 2.4 Hz, 1H), 3.97 (t, *J* = 8.8, 1H) 3.94 (t, *J* = 6.5, 2H), 3.75 (dd, *J* = 8.8, 2.4 Hz, 1H), 2.96 (t, *J* = 6.5 Hz, 2H), 2.80 (s, 3H), 2.78 (s, 3H), 1.06 (s, 9H).

### EXAMPLE 46 (+/-)-1-(4-((4'-(2-Hydroxyethyl)-3-methyl-[1,1'-biphenyl]-4-yl)diazenyl)-2-methylphenoxy)-3,3-dimethylbutan-2-ol ((+/-)-Ib-11)

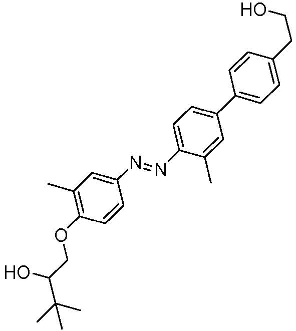

According to method D, from 4.0 mg of **((+/-)-la-11)** in THF (0.01 M), 2.4 mg of **((+/-)-Ib-11)** (35% yield) were obtained, once it was purified by reverse phase.

¹H NMR (400 MHz, CDCl₃) δ 7.84 (dd, *J* = 8.6, 2.0 Hz, 1H), 7.81 (d, *J =* 2.0 Hz, 1H), 7.71 (d, *J =* 8.4 Hz, 1H), 7.63 (d, *J* = 8.2 Hz, 2H), 7.57 (d, *J* = 2.0 Hz, 1H), 7.51 (dd, *J* = 8.4, 2.0 Hz, 1H), 7.35 (d, *J* = 8.2 Hz, 2H), 6.98 (d, *J =* 8.6 Hz, 1H), 4.24 (dd, *J =* 9.0, 2.6 Hz, 1H), 4.02 (t, *J* = 9.0 Hz, 1H), 3.95 (t, *J =* 6.5 Hz, 2H), 3.80 (dd, *J =* 9.0, 2.6 Hz, 1H), 2.96 (t, *J =* 6.5 Hz, 2H), 2.80 (s, 3H), 2.36 (s, 3H), 1.08 (s, 9H).

### Photochemistry

The photoisomerization properties of the azobenzene-based molecules developed in this study were studied using spectroscopic methods. Indeed, azobenzene moiety is known to exist in two main isomeric forms, trans and cis, with significantly different absorbance spectra at the UV-visible range. The distinct spectroscopic properties between the cis and the trans allows for the stabilization of different photoisomeric proportions upon illumination reaching a wavelength-dependent photostationary state. By introducing modifications in the azobenzene rings, the photochemical properties might be tuned. Therefore, in photopharmacology light-regulated drugs containing the azobenzene moiety must be characterized to determine the optimal photoisomerization wavelengths and the relaxation time. These photochemical properties will ultimately define the level of control of the biological system that can be exerted.

The UV-Vis absorbance spectra of all molecules were measured in the dark and after application of light with different wavelengths ranging from 365 nm to 770 nm. The resulting spectrum in the dark corresponding to the trans configuration has a maximum peak at 355 nm. On the other hand, violet/UVA/UVB light bellow 385 nm efficiently promoted photoisomerization, which consequently produced a decrease of the π-π* band and a significant increase of the n-π* band around 450 nm. Back-isomerization to the most stable trans configuration was achieved by blue to yellow illumination above 435 nm. Our results suggest very efficient interconversion between the two configurations and the absence of photodegradation.

### Photopharmacology

The activity of all molecules was evaluated using a luminescent proximity homogeneous assay (AlphaScreen^{®}, PerkinElmer^{™}) to study interactions between purified VDR, RxR and a peptide of the transcriptional coactivator TRAP220. This method allowed us to monitor the interaction between TRAP220 and the activated VDR-RxR heterodimer. Both the activation of VDR and the VDR-RxR heterodimer formation are necessary to promote the whole complex association. This system efficiently includes conformational events of the receptor upon activation that go beyond a simple binding measurement.

The library of compounds was first tested at 10 uM concentration in the dark and after illumination with light at 365 nm. We subsequently performed dose-concentration experiments of hits in which the EC₅₀ and the maximum response (Eₘₐₓ) could be accurately determined. Each experiment was repeated three times and dose-reponse data was fitted using the log (agonist) vs. response (three parameters) function.

**Table 1.- Pharmacological data of VDR agonists under illumination:**

| **Example.** | **VDR ligand** | **Activity (pEC₅₀)** | |
|---|---|---|---|
| | | **dark** | **light** |
| **LSN** | - | +++ | +++ |
| **1** | **(+/-)**-Ia-1 | + | +++ |
| **2** | (-)-Ia-1a | + | +++ |
| **3** | (+)-Ia-1 | + | +++ |
| **4** | **(+/-)**-Ia-2 | + | ++ |
| **5** | **(+/-)**-Ia-3 | + | +++ |
| **6** | **(+/-)**-Ia-4 | + | ++ |
| **7** | **(+/-)**-Ic-1 | + | +++ |
| **8** | (-)-Ic-1 | + | +++ |
| **9** | (+)-Ic-1 | + | +++ |
| **10** | **(+/-)**-Ic-2 | ++ | ++ |
| **11** | **(+/-)**-Ic-3 | + | +++ |
| **12** | **(+/-)**-Ic-4 | + | +++ |
| **13** | **(+/-)**-Ib-1 | + | +++ |
| **14** | (-)-Ib-1 | + | ++ |
| **15** | (+)-Ib-1 | + | ++ |
| **16** | **(+/-)**-Ib-2 | + | +++ |
| **17** | (-)-Ib-2 | + | ++ |
| **18** | (+)-Ib-2 | + | ++ |
| **19** | **(+/-)**-Ib-3 | + | +++ |
| **20** | **(+/-)**-Ib-4 | + | ++ |
| **21** | Id-1 | + | + |
| **22** | Id-2 | + | ++ |
| **23** | Ie-1 | + | ++ |
| **24** | Ie-2 | + | + |
| **25** | Ie-3 | + | +++ |
| **26** | Ie-4 | + | ++ |
| **34** | **(+/-)**-Ic-6 | + | ++ |

| | | | |
|---|---|---|---|
| Legend: +++: pEC50 > 6.0 ++: 5 < pEC50 < 6.0 +: pEC50 < 5 | | | |

## Claims

1. A compounds of formula (I): 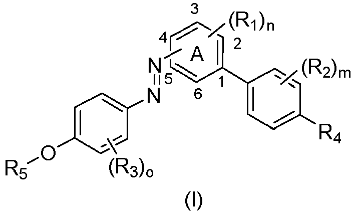 or a pharmaceutical salt thereof, wherein:
the azo group (-N=N-) is linked to any of the available positions 4- or 5- of the benzene ring A;
each of n, m and o independently represents an integer from 0 to 2;
each of R₁, R₂ and R₃ independently represents halogen, -C₁-C₄ alkyl, -OR₆, -OCF₃, - CF₃, -N(R₆)₂ or -NO₂;
R₄ represents -(C₁-C₄ alkyl)ₚOR₇, -(C₁-C₄ alkyl)_{q}COOR₈, -(C₁-C₄ alkyl)ᵣCONR₉R₁₀, each of p, q and r independently represents an integer having a value of from 0 to 3;
R₅ represents a linear or branched -C₂-C₇-alkyl group substituted with at least one hydroxyl group;
each R₆ independently represents H or -C₁-C₄ alkyl; and
each R₇, R₈, R₉ and R₁₀ independently represents H or -C₁-C₄-alkyl, wherein said -C₁-C₄-alkyl is optionally substituted by 1 to 3 groups independently selected from the group consisting of -OR₆, -N(R₆)₂, -COOR₆ or -CON(R₆)₂ ,
or, alternatively, when any one of R₇, R₈, R₉ and R₁₀ is a -C₁-C₄-alkyl substituted by two adjacent groups -OR₆ both of the -OR₆ groups can be bonded to form an acetal group.

2. The compound of formula (I) according to claim 1, wherein the azo group (-N=N-) is linked to position 5- of the benzene ring A.

3. The compound of formula (I) according to any of claims 1 or 2, wherein each of n, m and o independently represents an integer having a value of 0 or 1.

4. The compound of formula (I) according to any of claims 1 to 3, wherein each of R₁, R₂ and R₃ independently represents a -C₁-C₄ alkyl, preferably each of R₁, R₂ and R₃ independently represents a methyl group.

5. The compound of formula (I) according to any of claims 1 to 4, wherein R₅ represents a 2-hydroxy-3,3-dimethylbutyl group.

6. The compound of formula (I) according to any of claims 1 to 5, wherein each of p, q and r independently represents an integer having a value of 0 or 1.

7. The compound of formula (I) according to any of claims 1 to 6, wherein R₄ represents -(C₁-C₄ alkyl) _{q}COOR₈.

8. The compound of formula (I) according to claim 7, wherein R₈ represents H.

9. The compound of formula (I) according to any of claims 1 to 8, wherein:
R₄ represents -(C₁-C₄ alkyl)_{q}COOR₈; and
q represents an integer having a value of from 0 to 1;
R₈ is selected from the group consisting of H and -C₁-C₄-alkyl, preferably H or methyl group.

10. The compound of formula (I) according to any one of claims 1 to 9, wherein:
R₄ is -COOR₈ wherein R₈ is selected from the group consisting of hydrogen or methyl.

11. The compound of formula (I) according to claim 10, wherein each of R₁, R₂ and R₃ independently represents hydrogen or methyl group, n, m and o independently take a value of 0 or 1 and R₅ represents a 2-hydroxy-3,3-dimethylbutyl group.

12. The compound of formula (I) according to claim 1, which is selected from:
Methyl (+/-)-3'-((4-(2-hydroxy-3,3-dimethylbutoxy)-2-methylphenyl)diazenyl)-[1,1'-biphenyl]-4-carboxylate ((+/-)-la-1);
(-)-(R)-Methyl 3'-((4-(2-hydroxy-3,3-dimethylbutoxy)-2-methylphenyl)diazenyl)-[1,1'-biphenyl]-4-carboxylate ((-)-la-1);
(+)-(S)-Methyl 3'-((4-(2-hydroxy-3,3-dimethylbutoxy)-2-methylphenyl)diazenyl)-[1,1'-biphenyl]-4-carboxylate ((+)-la-1);
(+/-)-1-(4-((4'-(hydroxymethyl)-[1,1'-biphenyl]-3-yl)diazenyl)-2-methylphenoxy)-3,3-dimethylbutan-2-ol ((+/-)-la-2);
(+/-)-Methyl 3'-((4-(2-hydroxy-3,3-dimethylbutoxy)-2-methylphenyl)diazenyl)-2-methyl-[1,1'-biphenyl]-4-carboxylate ((+/-)-la-3);
(+/-)-Methyl 3'-((4-(2-hydroxy-3,3-dimethylbutoxy)-3-methylphenyl)diazenyl)-2-methyl-[1,1'-biphenyl]-4-carboxylate ((+/-)-la-4);
(+/-)-3'-((4-(2-Hydroxy-3,3-dimethylbutoxy)-2-methylphenyl)diazenyl)-[1,1'-biphenyl]-4-carboxylic acid ((+/-)-lc-1);
*(-)-(R)* 3'-((4-(2-Hydroxy-3,3-dimethylbutoxy)-2-methylphenyl)diazenyl)-[1,1'-biphenyl]-4-carboxylic acid((-)-Ic-1);
(+)-(S) 3'-((4-(2-Hydroxy-3,3-dimethylbutoxy)-2-methylphenyl)diazenyl)-[1,1'-biphenyl]-4-carboxylic acid ((+)-lc-1);
(+/-)-3'-((4-(2-Hydroxy-3,3-dimethylbutoxy)-3-methylphenyl)diazenyl)-[1,1'-biphenyl]-4-carboxylic acid ((+/-)-lc-2);
(+/-)-3'-((4-(2-Hydroxy-3,3-dimethylbutoxy)-2-methylphenyl)diazenyl)-2-methyl-[1,1'-biphenyl]-4-carboxylic acid ((+/-)-Ic-3);
(+/-)-3'-((4-(2-Hydroxy-3,3-dimethylbutoxy)-3-methylphenyl)diazenyl)-2-methyl-[1,1'-biphenyl]-4-carboxylic acid ((+/-)-Ic-4);
(+/-)-1-(4-((4'-(Hydroxymethyl)-[1,1'-biphenyl]-3-yl)diazenyl)-3-methylphenoxy)-3,3-dimethylbutan-2-ol ((+/-)-Ib-1);
(-)-1-(4-((4'-(Hydroxymethyl)-[1,1'-biphenyl]-3-yl)diazenyl)-3-methylphenoxy)-3,3-dimethylbutan-2-ol ((-)-Ib-1);
(+)-1-(4-((4'-(Hydroxymethyl)-[1,1'-biphenyl]-3-yl)diazenyl)-3-methylphenoxy)-3,3-dimethylbutan-2-ol ((+)-lb-1);
(+/-)-1-(4-((4'-(Hydroxymethyl)-[1,1'-biphenyl]-3-yl)diazenyl)-2-methylphenoxy)-3,3-dimethylbutan-2-ol ((+/-)-lb-2);
(-)-1-(4-((4'-(Hydroxymethyl)-[1,1'-biphenyl]-3-yl)diazenyl)-2-methylphenoxy)-3,3-dimethylbutan-2-ol ((-)-Ib-2);
(+)-1-(4-((4'-(Hydroxymethyl)-[1,1'-biphenyl]-3-yl)diazenyl)-2-methylphenoxy)-3,3-dimethylbutan-2-ol ((+)-lb-2);
(+/-)-1-(4-((4'-(Hydroxymethyl)-2'-methyl-[1,1'-biphenyl]-3-yl)diazinyl)-3-methylphenoxy)-3,3-dimethylbutan-2-ol ((+/-)-lb-3);
(+/-)-1-(4-((4'-(Hydroxymethyl)-2'-methyl-[1,1'-biphenyl]-3-yl)diazenyl)-2-methylphenoxy)-3,3-dimethylbutan-2-ol ((+/-)-lb-4);
Methyl-(3'-(4-(2-hydroxy-3,3-dimethylbutoxy)-3-methylphenyl)diazenyl)-[1,1'-biphenyl]-4-carbonyl)-L-serinate (Id-1);
(3'-(4-(2-Hydroxy-3,3-dimethylbutoxy)-3-methylphenyl)diazenyl)-[1,1'-biphenyl]-4-carbonyl)-L-serine) (Id-2);
1-(4-((4'-(((2,2-dimethyl-1,3-dioxolan-4-yl)methoxy)methyl)-[1,1'-biphenyl]-3-yl)diazinyl)-3-methylphenoxy)-3,3-dimethylbutan-2-ol (le-1);
1-(4-((4'-(((2,2-dimethyl-1,3-dioxolan-4-yl)methoxy)methyl)-[1,1'-biphenyl]-3-yl)diazenyl)-2-methylphenoxy)-3,3-dimethylbutan-2-ol (le-2);
3-((3'-((4-(2-hydroxy-3,3-dimethylbutoxy)-2-methylphenyl)diazenyl)-[1,1'-biphenyl]-4-yl)methoxy)propane-1,2-diol (le-3);
3-((3'-((4-(2-hydroxy-3,3-dimethylbutoxy)-3-methylphenyl)diazenyl)-[1,1'-biphenyl]-4-yl)methoxy)propane-1,2-diol (le-4);
(+/-)-Ethyl 4'-((4-(2-hydroxy-3,3-dimethylbutoxy)-2-methylphenyl)diazenyl)-[1,1'-biphenyl]-4-carboxylate ((+/-)-la-5);
(+/-)-Ethyl 4'-((4-(2-hydroxy-3,3-dimethylbutoxy)-3-methylphenyl)diazenyl)-[1,1'-biphenyl]-4-carboxylate ((+/-)-la-6);
(+/-)-Methyl 4'-((4-(2-hydroxy-3,3-dimethylbutoxy)-2-methylphenyl)diazenyl)-2-methyl-[1,1'-biphenyl]-4-carboxylate ((+/-)-la-7);
(+/-)-Ethyl 4'-((4-(2-Hydroxy-3,3-dimethylbutoxy)-2-methylphenyl)diazenyl)-3'-methyl-[1,1'-biphenyl]-4-carboxylate ((+/-)-la-8);
(+/-)-Ethyl 4'-((4-(2-hydroxy-3,3-dimethylbutoxy)-3-methylphenyl)diazenyl)-3'-methyl-[1,1'-biphenyl]-4-carboxylate ((+/-)-la-9);
(+/-)-4'-((4-(2-hydroxy-3,3-dimethylbutoxy)-2-methylphenyl)diazenyl)-[1,1'-biphenyl]-4-carboxylic acid ((+/-)-lc-5);
(+/-)-4'-((4-(2-Hydroxy-3,3-dimethylbutoxy)-3-methylphenyl)diazenyl)-[1,1'-biphenyl]-4-carboxylic acid ((+/-)-Ic-6);
(+/-)-4'-((4-(2-Hydroxy-3,3-dimethylbutoxy)-2-methylphenyl)diazenyl)-3'-methyl-[1,1'-biphenyl]-4-carboxylic acid ((+/-)-Ic-7);
(+/-)-4'-((4-(2-Hydroxy-3,3-dimethylbutoxy)-3-methylphenyl)diazenyl)-3'-methyl-[1,1'-biphenyl]-4-carboxylic acid ((+/-)-Ic-8);
(+/-)-1-(4-((4'-(Hydroxymethyl)-[1,1'-biphenyl]-4-yl)diazenyl)-3-methylphenoxy)-3,3-dimethylbutan-2-ol ((+/-)-Ib-5);
(+/-)-1-(4-((4'-(Hydroxymethyl)-[1,1'-biphenyl]-4-yl)diazenyl)-2-methylphenoxy)-3,3-dimethylbutan-2-ol ((+/-)-Ib-6);
(+/-)-1-(4-((4'-(Hydroxymethyl)-2'-methyl-[1,1'-biphenyl]-4-yl)diazenyl)-3-methylphenoxy)-3,3-dimethylbutan-2-ol ((+/-)-lb-7);
(+/-)-1-(4-((4'-(Hydroxymethyl)-3-methyl-[1,1'-biphenyl]-4-yl)diazenyl)-2-methylphenoxy)-3,3-dimethylbutan-2-ol ((+/-)-lb-8);
(+/-)-1-(4-((4'-(Hydroxymethyl)-3-methyl-[1,1'-biphenyl]-4-yl)diazenyl)-3-methylphenoxy)-3,3-dimethylbutan-2-ol ((+/-)-lb-9);
(+/-)-2-(4'-((4-(2-Hydroxy-3,3-dimethylbutoxy)-2-methylphenyl)diazenyl)-3'-methyl-[1,1'-biphenyl]-4-yl)acetic acid ((+/-)-Ic-9);
(+/-)-2-(4'-((4-(2-Hydroxy-3,3-dimethylbutoxy)-3-methylphenyl)diazenyl)-3'-methyl-[1,1'-biphenyl]-4-yl)acetic acid ((+/-)-Ic-10);
(+/-)-Methyl 2-(4'-((4-(2-hydroxy-3,3-dimethylbutoxy)-2-methylphenyl)diazenyl)-3'-methyl-[1,1'-biphenyl]-4-yl)acetate (I(+/-)-a-10);
(+/-)-Methyl 2-(4'-((4-(2-hydroxy-3,3-dimethylbutoxy)-3-methylphenyl)diazenyl)-3'-methyl-[1,1'-biphenyl]-4-yl)acetate ((+/-)-la-11);
(+/-)-1-(4-((4'-(2-hydroxyethyl)-3-methyl-[1,1'-biphenyl]-4-yl)diazinyl)-3-methylphenoxy)-3,3-dimethylbutan-2-ol ((+/-)-lb-10); and
(+/-)-1-(4-((4'-(2-Hydroxyethyl)-3-methyl-[1,1'-biphenyl]-4-yl)diazenyl)-2-methylphenoxy)-3,3-dimethylbutan-2-ol ((+/-)-lb-11).

13. A pharmaceutical composition which comprises the compound of formula (I) according to any of claims 1 to 12 or a pharmaceutically acceptable salt thereof and one or more pharmaceutically acceptable excipients

14. A compound of formula (I) according to any of claims 1 to 12 or a pharmaceutically acceptable salt thereof for use as a medicament.

15. A compound of formula (I) according to any of claims 1 to 12 or a pharmaceutically acceptable salt thereof for use in the treatment of a disease selected from skin diseases, immune diseases, cancer, disorders of calcium metabolism, neuromuscular disorders, and cardiovascular diseases.
